(19) Europäisches Patentamt
European Patent Office
Office européen des brevets

(11) Publication number: **0 217 804 B1**

(12) **EUROPEAN PATENT SPECIFICATION**

(45) Date of publication of patent specification: **02.10.91**

(51) Int. Cl.5: **C07K 7/06, C07C 69/96**

(21) Application number: **86900104.0**

(22) Date of filing: **05.12.85**

(86) International application number:
**PCT/EP85/00667**

(87) International publication number:
**WO 86/03495 (19.06.86 86/13)**

The file contains technical information submitted after the application was filed and not included in this specification

(54) **ANALOGS OF SUBSTANCES P.**

(30) Priority: **11.12.84 IT 2399384**

(43) Date of publication of application:
**15.04.87 Bulletin 87/16**

(45) Publication of the grant of the patent:
**02.10.91 Bulletin 91/40**

(84) Designated Contracting States:
**AT BE CH DE FR GB LI LU NL SE**

(56) References cited:
**DE-A- 2 626 346**
**DE-A- 2 720 290**
**US-A- 3 862 114**
**US-A- 4 059 693**

**Chemical Abstracts, vo. 92, 1980, Columbus, Ohio (US), page 706, abstract 181680q**

(73) Proprietor: **ENICHEM S.p.A.**
**Via Medici del Vascello 26**
**I-20138 Milano(IT)**

(72) Inventor: **BONELLI, Fabio**
**Via Don Carlo Gnocchi, 10**
**I-00166 Rome(IT)**
Inventor: **FARINA, Antonio**
**Via Anagnina, 322**
**I-00173 Rome(IT)**
Inventor: **PESSI, Antonello**
**Via Massaciuccoli, 19**
**I-00199 Rome(IT)**
Inventor: **SQUADRINI, Fabio**
**Via A. Guarnieri, 4**
**I-63023 Fermo, Ascoli Piceno(IT)**
Inventor: **VERDINI, Antonio, Silvio**
**Via S. Martino, 12**
**I-00015 Monterotondo, Rome(IT)**
Inventor: **VISCOMI, Giuseppe, Claudio**
**Via 8 Maggio, 23**
**I-00015 Monterotondo, Rome(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

(74) Representative: **Roggero, Sergio et al**
**Ing. Barzanò & Zanardo Milano S.p.A. Via Borgonuovo 10**
**I-20121 Milano(IT)**

## Description

This invention relates to analogs of "Substance P", at least one aminoacid of which bears, chemically bonded, in the case of an acidic aminoacid, a methoxy-(ethoxy)$_n$ ethoxy substituent, and/or, in the case of a basic aminoacid, a methoxy(ethoxy)$_n$ethoxycarbonyl substituent.

Substance P, or SP for short, is an undecapeptide represented by the sequence:
H-Arg-Pro-Lys-Pro-Gln-Gln-Phe-Phe-Gly-Leu-Met-$NH_2$
and is responsible for a number of pharmacological effects.

In the CNS, it is active as a pain threshold modulator, as a neurone depolarizer, and as a locomotion improver. In the gastroenteric tract, it induces peristaltic reflexes, improves the tonus and is active in tissue contraction.

In the cardiovascular system, it induces both hypotension and vasodilation and, in the secretory system, it enhances salivation and prolactin release.

Each and every biological effect can be attributed to a well defined segment of the SP sequence.

Sialogic activity, for example, is brought about by the entire sequence, the depolarization of motoneurons is due to the $SP_{5-11}$ and $SP_{6-11}$ segments, the spasmogenic activity to the $SP_{3-11}$ and $SP_{4-11}$ segments, and the analgesic activity to the $SP_{1-7}$ segment.

Such a wide spectrum of pharmacological activities may suggest that a number of active nuclei subsist in the SP molecule, and that they are set free by specific SP-enzymes, wherefore the several SP portions so produced combine with the respective receptors and bring about a number of different effects.

A decisive parameter to assess the pharmacological activity of SP analogs is their distribution in the living tissues: if such distribution is modified, specific activities may be obtained, either by selecting tissues containing several receptors, or by selecting tissues which contain certain enzymes, or, also, by selecting tissues which do not contain specific proteolytic enzymes, so that the analog can be stored and released slowly.

Generally, the distribution of the analogs in the living tissues can be modified by bonding the molecules of interest to organic hydrophilic and/or hydrophobic organic groups.

It is likewise known that the solubility of the SP can be modified by groups such as the residue of gluconic acid (Bienert,M. et al, "Peptides", (1982) New York, Walther De Gruyher & Co., (1982) and (1983) page 517), and the N 1-(9-adenyl)- -D-furanosyl and N 1-( -hypoxantyl) -D-ribofuranosyl residues (Hirotoshi, M. et al, "Peptide Chemistry", (1980) page 175 (1981). It is known, furthermore, that the pharmacologocal activity of proteins and enzymes can be enhanced by chemically bonding them to to polymeric chains such as polyvinyl macromolecules, or polyethylene glycol chains: the latter are not immunogenic and are _in vivo_ compatible, so that they can be harmlessly administered for therapeutical use.

The present invention, therefore, provides

Analogs of "Substance P" represented by the general formula:

$R\text{-}R^5\text{-}R^4\text{-}R^3\text{-}R^2\text{-}R^1\text{-}NH_2$ (I)

at least one amino acid of which bears, chemically bonded, in the case of an acidic amino acid, a methoxy-(ethoxy)$_n$ethoxy substituent of general formula:

$CH_3\text{-}O\text{-}(CH_2\text{-}CH_2\text{-}O)_n\text{-}CH_2\text{-}CH_2\text{-}O\text{-}$ (II) (MEE)

with _n_ = 0, 1, 2, 3,
and/or, in the case of a basic amino acid, a methoxy(ethoxy)$_n$ethoxycarbonyl substituent of the general formula:

$CH_3\text{-}O\text{-}(CH_2\text{-}CH_2\text{-}O\text{-})_n\text{-}CH_2\text{-}CH_2\text{-}O\text{-}CO\text{-}$ (III)(MEEEOC)

with _n_ = 0, 1, 2, 3,
wherein:

$R^1$ is a residue of methionine, methionine sulphoxide, methionine sulphone, or seleno-methionine;
$R^2$ a residue of leucine, norleucine, or isoleucine;
$R^3$ is a residue of glycine;
$R^4$ is a residue of phenylalanine, tryptophan, or tyrosine;
$R^5$ is a residue of phenylalanine, tyrosine, 4-chlorophenylalanine, o-benzyltyrosine, and acetyl-, cyclopentyl-, tert.butyloxycarbonyl- and 4-hydroxyphenylacetyl derivatives thereof, and R is selected

from among:

a) a group having the general formula (III) (MEEEOC) wherein n = 0, 1, 2, 3;

b) a derivative of an amino acid having the general formula:

P-G (IV)

wherein G is a residue having the general formula:

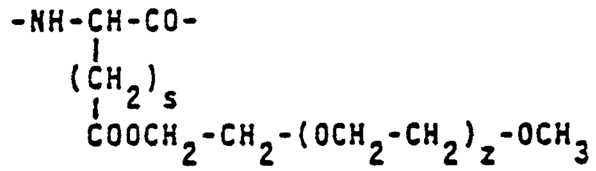

(V)

in which s is 1 or 2, and z is 0, 1, 2, 3, and P is a hydrogen atom, a linear or a branched aliphatic alkyl group having from 1 to 6 carbon atoms, or a linear or a branched, saturated- or unsaturated-chain aliphatic acyl group selected from among formyl, acetyl, propionyl, n-butyryl, isobutyryl, n-valeryl, isovaleryl, hexanoyl, isohexanoyl, heptanoyl, octanoyl, crotonoyl, methacryloyl, acryloyl, or a substituted acyl group selected from among: hydroxyacetyl, 2-hydroxypropanoyl, 2-hydroxypropanoyl, 3-hydroxypropanoyl, aminoacetyl, 4-hydroxyphenylacetyl, 4-hydroxyphenylpropanoyl, 2-aminopropanoyl, 3-aminopropanoyl, o-ethylmalonyl, ethoxyformyl, methoxyformyl, 3-methoxypropanoyl, 3-ethoxypropanoyl, chloroacetyl, dichloroacetyl, 2-chloropropanoyl, 3-chloropropanoyl, 2,3-dichloropropanoyl, bromoacetyl, 4-hydroxy-3,5-diiodophenylacetyl, 3-oxobutyryl, 3-oxovaleryl, methylthioacetyl, 3-methylthiopropanoyl, ethylthioacetyl, 3-ethylthiopropanoyl, nicotinoyl, 4-aminobutyryl, $N^{\alpha}$-[1-(9-adenyl)-$\beta$-D-ribofuranuronosyl)], $N^{\alpha}$-[(1-(9-hypoxanthyl)-$\beta$-D-ribofuranuronosyl)], or a group selected from among benzyloxycarbonyl, tert.butyloxycarbonyl, tert.amiloxycarbonyl, isobornyloxycarbonyl, adamantyloxycarbonyl, chloro- or nitro substituted benzyloxycarbonyl;

c) an amino acid derivative having the general formula:

$CH_3\text{-}O(CH_2\text{-}CH_2\text{-}O)_n\text{-}CH_2\text{-}CH_2\text{-}O\text{-}CO\text{-}R^6$ (VI)

wherein n is 0, 1, 2, 3, and $R^6$ is a residue of glutamine and the MEEEOC derivative thereof, or a residue of formula (V) wherein s and z are as defined above;

d) a derivative of a dipeptidic segment having the general formula:

$CH_3\text{-}O\text{-}(CH_2\text{-}CH_2\text{-}O)_n\text{-}CH_2\text{-}CH_2\text{-}O\text{-}CO\text{-}R^8\text{-}R^7$ (VII)

wherein n is 0, 1, 2, 3, $R^7$ is a residue of glutamine or a residue of formula (V), wherein s and z are as defined above, and $R^8$ is a residue of glutamine or the MEEEOC derivative thereof, or a residue having the general formula:

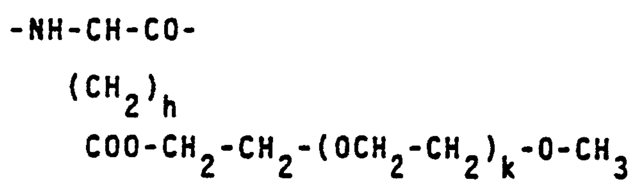

(VIII)

wherein h is 1 or 2, and k is 0, 1, 2, 3;

e) a derivative of a dipeptidic segment having the general formula:

$P\text{-}R^{10}\text{-}R^9$ (IX)

wherein P has the previously disclosed meaning, $R^9$ is a residue of formula (V), and $R^{10}$ is a residue of glutamine or the MEEEOC derivative thereof, or a derivative of formula (VIII), or wherein $R^{10}$ is a residue of formula (V) and $R^9$ is a residue of glutamine or the MEEEOC derivative thereof, or a residue of formula (VIII);

f) a derivative of a tripeptidic segment having the general formula:

$CH_3$-O-($CH_2$-$CH_2$-O)$_n$-$CH_2$-$CH_2$-O-CO-$R^{11}$-$R^8$-$R^7$ (X)

wherein n, $R^8$ and $R^7$ have the previously indicated meanings, and
$R^{11}$ is a residue of proline;
g) a derivative of a tripeptidic segment having the general formula:

P-$R^{11}$-$R^9$-$R^{10}$ (XI)

wherein P, $R^9$, $R^{10}$ and $R^{11}$ have the previously disclosed meanings;
h) a tetrapeptidic derivative having the general formula:

$CH_3$-O($CH_2$-$CH_2$-O-)$_n$-$CH_2$-$CH_2$-O-CO-$R^{12}$-$R^{11}$-$R^8$-$R^7$ (XII)

wherein n, $R^7$ $R^8$, and $R^{11}$ have the previously disclosed meanings, and $R^{12}$ is a residue of lysine or the MEEEOC derivative thereof, or is a residue having the general formula:

-NH-CH-CO-
($CH_2$)$_j$
NH-CO-O-$CH_2$-$CH_2$-(O-$CH_2$-$CH_2$)$_i$-$OCH_3$ (XIII)

wherein i is 0, 1, 2, 3, and j is 1, 2, 3, 4;
i) a tetrapeptidic derivative having the general formula:

P-$R^{12}$-$R^{11}$-$R^{10}$-$R^9$ (XIV)

wherein P, $R^9$, $R^{10}$ $R^{11}$ and $R^{12}$ have the previously disclosed meanings;
j) a pentapeptide derivative having the general formula:

$CH_3$-O($CH_2$-$CH_2$-O-)$_n$-$CH_2$-$CH_2$-O-CO-$R^{13}$-$R^{12}$-$R^{11}$-$R^8$-$R^7$ (XV)

wherein n, $R^7$, $R^8$, $R^{11}$, and $R^{12}$ have the previously disclosed meanings, and $R^{13}$ is a residue of proline;
k) a pentapeptide derivative having the general formula:

P-$R^{13}$-$R^{12}$-$R^{11}$-$R^{10}$-$R^9$ (XVI)

wherein P, $R^9$, $R^{10}$, $R^{11}$, $R^{12}$ and $R^{13}$ have the previously disclosed meanings;
l) a hexapeptide derivative having the general formula:

$CH_3$-O($CH_2$-$CH_2$-O)$_n$-$CH_2$-$CH_2$-O-CO-$R^{14}$-$R^{13}$-$R^{12}$-$R^{11}$-$R^8$-$R^7$ (XVII)

wherein n, $R^7$, $R^8$, $R^{11}$, $R^{12}$ and $R^{13}$ have the previously disclosed meanings, and $R^{14}$ is a residue of arginine and the MEEEOC derivative thereof, or
m) a hexapeptidic derivative having the general formula:

P-$R^{14}$-$R^{13}$-$R^{12}$-$R^{11}$-$R^{10}$-$R^9$ (XVIII)

wherein P, $R^9$, $R^{10}$, $R^{11}$, $R^{12}$, $R^{13}$ and $R^{14}$ have the previously disclosed meanings.
Preferred are those analogs of Substance P wherein:
$R^1$ is a residue of methionine, methionine sulphoxide, methionine sulphone or seleno-methionine;
$R^2$ is a residue of leucine, norleucine, or isoleucine;
$R^3$ is a residue of glycine;

$R^4$ is a residue of phenylalanine, tryptophan, or tyrosine, and

$R^5$ is a residue of phenylalanine, tyrosine, 4-chlorophenylalanine, o-benzyltyrosine, and acetyl-, cyclopentyl-, tert.butyloxycarbonyl-, and 4-hydroxyphenylacetyl derivatives thereof.

Other preferred embodiments are analogs of Substance P wherein:

R is selected from among:

a) a group of formula (III) (MEEEOC);

b) an amino acid derivative of formula (IV) wherein G is a residue of formula (V), and P, s, and z have the previously disclosed meanings;

c) an amino acid derivative of formula (VI), wherein n and $R^6$ have the previously disclosed meanings, or a residue of formula (V), wherein s and z have the previously disclosed meanings;

d) a derivative of a dipeptidic segment of formula (VII), wherein n has the previously disclosed meaning and $R^7$ is a residue of glutamine, or a residue of formula (V) wherein s and z have the previously disclosed meanings and $R^8$ is a residue of glutamine or the MEEEOC derivative thereof, or a residue of formula (VIII) wherein h and k have the previously disclosed meanings;

e) a derivative of a dipeptidic segment of formula (IX), wherein P has the previously disclosed meaning and $R^9$ is a residue of formula (V) wherein s and z have the previously disclosed meanings, and $R^{10}$ is a residue of glutamine or the MEEEOC derivative thereof, or a derivative of formula (VIII) wherein h and k have the previously disclosed meanings, or $R^{10}$ is a residue of formula (V) wherein s and z have the previously disclosed meanings, and $R^9$ is a residue of glutamine or the MEEEOC derivative thereof, or a residue of formula (VIII) wherein h and k have the previously disclosed meanings;

f) a derivative of a tripeptidic segment of formula (X) wherein n, $R^7$, $R^8$ and $R^{11}$ have the previously disclosed meanings;

g) a derivative of a tripeptidic segment of formula (XI) wherein P, $R^9$, $R^{10}$ and $R^{11}$ have the previsouly disclosed meanings;

h) a hexapeptidic derivative of formula (XVII) wherein n, $R^7$, $R^8$, $R^{11}$, $R^{13}$ and $R^{14}$ have the previously disclosed meanings, and

i) a hexapeptidic segment of formula (XVIII) wherein P, $R^9$, $R^{10}$, $R^{11}$, $R^{12}$, $R^{13}$ and $R^{14}$ have the previously disclosed meanings.

The groups represented by the formulae (II) and (III) show a wide solubility spectrum both in polar as well as in apolar solvents, thus favouring the diffusion in vivo, both into hydrophilic and in lipophilic zones, of SP's derivatives into which they are incorporated.

The groups represented by formula (II) are introduced into SP's molecule as esters of residues of glutamic or aspartic acid, by replacing the natural residue of glutamine or aspartic acid. The esters of glutamic or aspartic acid are synthesized by using derivatives of the two aminoacid suitably protected at $-NH_2$ and at -COOH groups, causing the free carboxy group to condensate with methoxy(ethoxy)$_n$ethanol in the presence of N,N-dicyclohexylcarbodiimide and N,N-dimethylaminopyridine. The derivatives containing the oxycarbonyl groups represented by the general formula (III) are obtained by means of the reaction of methoxy(ethoxy)$_n$ethylchlorocarbonate, with formula $CH_3-O(CH_2-CH_2-O)_nCH_2-CH_2-O-CO-Cl$ with the selected aminoacid, after having suitably protected all other reactive functional group present.

The compound methoxy(ethoxy)$_n$ethyl-chlorocarbonateis prepared from methoxy(ethoxy)$_n$ethanol, previously distilled and cooled at -10°C, in the presence of phosgene, under strong stirring.

At the end of the reaction, the solution is brought to room temperature. The process is thereafter continued by evaporating off the excess of phosgene and hydrogen chloride formed from the reaction vessel, by means of a dry nitrogen flow.

The product so obtained is recovered from the reaction medium by fractional distillation.

The incorporation of aminoacidic derivatives containing the groups represented by the general formulae II and III in the polypeptide chain can be affected both by condensation with N,N'-dicyclohexylcarbodiimide/hydroxybenzotriazole, and by activation of -COOH via mixed anhydrides, or via symmetrical anhydrides, or via active esters.

The analogs represented by the formula I have the aminoacids with L-configuration.

The identity of the products obtained is demonstrated by means of resonance spectroscopic analysis and analysis of aminoacids.

The purity of the compounds is demonstrated by means of High Pressure Reverse Phase (RP-hplc) chromatographic analysis, using 0.01 N ammonium acetate/MeOH as the elutant, or thin-layer chromatographic analysis on silica gel, using the following elutant systems: n.butanol/acetic acid/water (4:1:1); chloroform/methanol/acetic acid (35:10:5).

In the description of the syntheses, abbreviations are used, which have the following meaning: THF = tetrahydrofuran, DMF = N,N-dimethylformamide, MeOH = methanol; EtOH = ethanol, EtAc = ethyl

acetate, TFA = trifluoroacetic acid, $Et_2O$ = diethyl ether, NMM = N-methylmorpholine, DCC = N,N'-dicyclohexylcarbodiimide; DCU = N,N'-dicyclohexylurea, HOBt = N-hydroxybenzotriazole, DMAP = N,N-dimethyl-p-aminopyridine, $NH_3$.HOBt = HOBt ammonium salt; BOC = tert. butyl-oxycarbonyl, Z = benzyloxycarbonyl, Fmoc = 9-fluorenyloxycarbonyl, Me = methyl, Et = ethyl, Su = succinimidyl, Bzl = benzyl, MEEEOC =
$CH_3-O(CH_2-CH_2-O)_2CH_2-CH_2-OCO-$,
OEEM =
$CH_3O-CH_2-CH_2O-CH_2-CH_2-O$,
HOSu = N-hydroxysuccinimide,and MEE = $CH_3-O(CH_2-CH_2-O)_NCH_2-CH_2-O$

Compositions useful in the pharmaceutical field are prepared, comprising a derivative of SP, or analogs of SP as previously defined, and a pharmaceutically acceptable diluent.

The said compositions may contain conventional excipients, and can be obtained by means of known techniques. Said compositions can be used in the treatment of acute and chronic hypertensions and, generally, under all those pathologic conditions as due to an insufficiency of SP or to an incapability to use SP. Said compositions may be moreover used in diagnostics, and in particular in the radio-immunological analysis of SP. The pharmacological activity of the compounds according to the present invention is verified by means of measurements of the contraction of isolated guinea-pig ileum, as described by Rossel et al. in "Substance P", U.S. Von Euler and B. Perow publ., Raven. Press, New York, 1977, page 83, and by evaluation in vivo of the main haemodynamic parameters, or by injection in vivo (0.4 mg/kg/l.v) in normotensive male rats; the results are reported in the following Table 1.

The following experimental examples are illustrative and not limitative of the invention itself.

Example 1

Synthesis of methoxy(ethoxy)$_2$ethyloxycarbonyl-glutaminyl-phenylalanyl-phenylalanyl-glycyl-leucyl-methioninamide MEEEOC-Gln-Phe-Phe-Gly-Leu-Met-$NH_2$

Synthesis of methyl ester of tert.butyl-oxycarbonyl-leucyl-methionine Boc-Leu-Met-OMe

1.0 Equivalents of Boc-Leu-OH are dissolved in anhydrous THF and to the solution, cooled at -15°C and maintained under a nitrogen atmosphere, 1.0 equivalents of NMM and 1.1 equivalents of isobutylchloroformate are added.

After two minutes, a solution is added, as obtained by dissolving 1.0 equivalents of HCl.H-Met-OMe and 1.0 equivalents of NMM in DMF. During the additions, the temperature is controlled not to rise above -10°C. After that the disappearing of HCl.H-Met-OMe as been verified, the reaction is stopped by evaporating the mixture to dryness, the residue is dissolved with EtAc and washed with a solution of bicarbonate at 5%, water, solution of citric acid at 5%, water.

The solution of EtAc is then dried over magnesium sulphate and the product is obtained by crystallization by means of a suitable addition of petroleum ether 30-50°C. Melting point = 102-104°C.

TABLE 1

| Substance | Contractive activity on isolated guinea-pig ileum | Reduction of arterial pressure in mmHg (diastolic-systolic) | Reduction of carotidal flow | Duration of effect |
|---|---|---|---|---|
| $SP_1$-11 | 1 | 30 - 55 | 40% | 25' |
| Meeeoc $Gln^6$ $SP_{6-11}$ | 10 | 50 - 50 | 43% | 50' |
| $Lys^3$(Meeeoc), $Glu^6$(OEEM) $SP_{1-11}$ | -- | 45 - 45 | 57% | 1 hour |

$[\alpha]_D^{22}$ = -36.1° (c = 1.0 in DMF).

Elemental analysis for $C_{17}H_{32}N_2O_5S$:

Theoretical: C, 54.23; H, 8.57; N, 7.44

Found : C, 54.10; H, 8.49; N, 7.39

The chromatographic analysis (tic and hplc) does not detect the presence of impurities, and $^1H$ N.M.R. spectrum confirms the molecule's structure.

Synthesis of tert.butyloxycarbonyl-leucyl-methioninamide Boc-Leu-Met-$NH_2$

1.0 Equivalents of Boc-Leu-Met-OMe are dissolved in anhydrous MeOH and into this solution, cooled at -5°C, anhydrous ammonia is flowed for 30 minutes.

The solution, contained in a tightly sealed vessel, is kept overnight at room temperature and the product in crystalline form is then obtained by means of the addition of a water volume of about 30% of MeOH volume. Melting point: 158-160°C.
$[\alpha]_D^{22}$ = -35.4° (c = 1.0 in DMF).

Elemental analysis for $C_{16}H_{31}N_3O_4S$:

Theoretical: C, 53.16; H, 7.33; N, 11.63

Found : C, 53.03; H, 7.23; N, 11.50

The chromatographic analysis (tlc and hplc) does not detect presence of impurities and the $^1H$ N.M.R. spectrum confirms the molecule's structure.

Synthesis of tert.butyloxycarbonyl-phenylalanyl-glycine methyl ester. Boc-Phe-Gly-OMe.

1.0 Equivalents of Boc-Phe-OH are dissolved in anhydrous THF and to the solution, cooled at -15°C and maintained under nitrogen atmosphere, 1.1 equivalents of NMM and 1.1 equivalents of isobutylchloroformate are added. After 2 minutes, 1.0 equivalents of HCl.H-Gly-OMe dissolved in anhydrous DMF and 1.0 equivalents of NMM are added. During the additions the temperature is controlled not to exceed -10°C.

After having verified the disappearing of HCl.H-Gly-OMe, the reaction is stopped by evaporating the solvent mixture to dryness; the residue is dissolved with EtAc and washed with sodium bicarbonate at 5%, water, citric acid at 5%, water. The organic solution is dried over magnesium sulphate; the product is obtained by crystallization from EtAc/ petroleum ether.
Melting point = 92-94°C
$[\alpha]_D^{22}$ = -11° (c = 0.5 in EtAc).

Elemental analysis $C_{17}H_{21}N_2O_5$:

Theoretical: C, 60.70; H, 7.19; N, 8.33

Found : C, 60.55; H, 6.88; N, 7.95.

The chromatographic analysis (tlc and hplc) does not reveal presence of impurities and the spectrum $^1N$ N.M.R. confirms the structure of the molecule.

Synthesis of tert.butyloxycarbonyl-phenylalanyl-phenylalanyl-glycine methyl ester. Boc-Phe-Phe-Gly-OMe.

1.0 Equivalents of Boc-Phe-OH are dissolved in anhydrous THF and to the solution cooled at -15°C and kept under nitrogen atmosphere 1.1 equivalents of NMM and 1.1 equivalents of isobutylchloroformate are added. After 2 minutes 1.0 equivalents of HCl.H-Phe-Gly-OMe (obtained by removing the tert.butyloxycarbonyl group from Boc-Phe-Gly-OMe with 4.5N HCl in EtAc) and 1.0 equivalents of NMM, dissolved in anhydrous DMF are added. During the addition the temperature is controlled not to rise over

-10 °C. After that the disappearing of HCl.H-Phe-Gly-OMe has been verified, the reaction is stopped, the volume of the solution is reduced by evaporation and the desired product is precipitated with an excess of water. The precipitate is washed with citric acid at 5%, water, sodium bicarbonate, water, and is dried over $P_2O_5$. The product is obtained by crystallization from DMF/$Et_2O$.
Melting point: 159-161 °C
$[\alpha]_D^{22}$ = -28 °C (c = 0.5 in DMF).

Elemental analysis for $C_{26}H_{33}N_3O_6$
Theoretical: C, 64.58; H, 6.88; N, 8.69
Found : C, 64.25; H, 6.50; N, 8.50

The chromatographic analysis (tlc and hplc) does not reveal presence of impurities and the ¹H N.M.R. spectrum confirms the identity of the molecule.

Synthesis of tert.butyloxycarbonyl-phenylalanyl-phenylalanyl-glycyl-leucyl-methioninamide Boc-Phe-Phe-Gly-Leu-Met-$NH_2$

1.0 Equivalents of Boc-Phe-Phe-Gly-OH (obtained by basic hydrolysis of Boc-Phe-Phe-Gly-OMe with 1.0 equivalents of 1M NaOH) are dissolved in anhydrous THF, and to the solution cooled at -15 °C and kept under nitrogen atmosphere, 1.1 equivalents of NMM and 1.1 equivalents of isobutylchloroformate are added. After 2 minutes, to the solution 1.0 equivalents of HCl-Leu-Met-$NH_2$ (obtained by removing the tert.butyloxycarbonyl group from Boc-Leu-Met-$NH_2$ with 4.5N HCl in EtAc) and 1.0 equivalents of NMM, dissolved in anhydrous DMF are added. During the additions the temperature is controlled not to rise above -10 °C.

The reaction is left overnight at room temperature; after that the reaction has been stopped and the solvents concentrated, the desired product is obtained by precipitation with an excess of water. The precipitate is washed with citric acid at 5%, water, sodium bicarbonate at 5%, water. The precipitate is dried over $P_2O_5$, dissolved in DMF, and precipitated with $Et_2O$.
Melting point = 223-227 °C.
$[\alpha]_D^{22}$ = -33 ° (c = 1 in DMF).

Elemental analysis for $C_{36}H_{52}N_6O_7S$
Theoretical: C, 60.65; H, 7.35; N, 11.79.
Found : C, 60.83; H, 7.30; N, 11.50.

The analysis of the aminoacids confirms the identity of the product; the chromatographic analysis (tlc and hplc) does not reveal presence of impurities.

Synthesis of methoxy(ethoxy)$_2$ethylchlorocarbonate:

$CH_3$-O($CH_2$-$CH_2$-O)$_2$$CH_2$-$CH_2$-O-CO-Cl

Through 1.0 equivalents of $CH_3$-O($CH_2$-$CH_2$-O)$_2$$CH_2$-$CH_2$OH, distilled and cooled at -10 °C, phosgene is made flow up to saturation. The temperature is maintained between -10 °C and -30 °C, and the solution is vigorously stirred.

After that the saturation has been reached, the solution is allowed to reach room temperature. The excess of phosgene is evaporated off and hydrogen chloride formed is removed by a stream of dry nitrogen. The last traces of gas are removed under vacuum and then, by fractional distillation in vacuo, the product desired is obtained as a colourless oil, which is stored over anhydrous sodium sulphate at -20 °C.

The I.R. analysis of obtained oil confirms the identity of the product.

Synthesis of methoxy(ethoxy)$_2$ethyloxycarbonyl-glutamine MEEEOC-Gln-OH

1.0 equivalents of H-Gln-OH are dissolved in a solution of 4N NaOH containing 1.0 equivalents of NaOH.

To this solution, cooled at 5° C, 1.1 equivalents of methoxy(ethoxy)$_2$ethylchlorocarbonate dissolved in a 4N NaOH solution, containing 1.2 equivalents of NaOH are slowly added.

After having verified the disappearing of H-Gln-OH used as the starting product, the solution is extracted twice with $Et_2O$, then acidified to Congo red with a 5N HCl solution, and is finally repeatedly extracted with EtAc. The organic fractions are combined, washed with water, dried over $MgSO_4$, and evaporated to dryness, a colourless oil being thus obtained. The desired product is obtained by ion-exchange chromatography, using a strong anionic resin (Merck N° 3) in $OH^-$ form, and using 0.01M $NH_4OH$ as the charge elutant, and 0.5M HCl as the elution solvent.

Elemental analysis for $C_{13}H_{24}N_2O_8$
Theoretical: C, 46.42; H, 7.19; N, 8.33
Found : C, 46.15; H, 6.99; N, 8.19.

The chromatographic analysis (tlc and hplc) does not reveal presence of impurities, the $^1H$ N.M.R. spectrum confirms the identity of the product.

Synthesis of methoxy(ethoxy)$_2$ethyl-oxycarbonyl-glutaminyl-phenylalanyl-phenylalanyl-glycyl-leucyl-methioninamide MEEEOC-Gln-Phe-Phe-Gly-Leu-Met-$NH_2$

1.0 Equivalents of MEEEOC-Gln-OH are dissolved in anhydrous THF and are cooled in an ice bath, and to the solution are then added, in the order, 1.1 equivalents of HOSu and 1,1 equivalents of DCC. After one hour, the solution is allowed to reach room temperature. After three hours the solution is filtered directly on a solution of 0.5 equivalents of HCl-Phe-Phe-Gly-Leu-Met-$NH_2$ (obtained by removing the tert.butyloxycarbonyl group of Bcc-Phe-Phe-Gly-Leu-Met-$NH_2$with 4.5N HCl in EtAc), and 0.5 equivalents of NMM in DMF. The solution is left overnight at room temperature, the reaction solvents are then partly evaporated, and by addition into a water excess a white precipitate is obtained, which is washed with citric acid at 5%, water, sodium bicarbonate at 5%, water. The desired product is obtained after drying over $P_2O_5$, dissolving in DMF and precipitation with $Et_2O$.
Melting point: 215-230° C
$[\alpha]_D^{22}$ = - 46.2 (c = 0.8 DMF).

Elemental analysis for $C_{44}H_{64}N_8O_{12}S$
Theoretical: C, 56.88; H, 6.94; N, 12.06
Found : C, 56.75; H, 6.90; N, 11.95

The chromatographic analysis (tlc and hplc) does not reveal presence of impurities, whilst the analysis of the aminoacids confirm the product's identity.

Example 2

Synthesis of tert.butyloxycarbonyl-glutamyl(γ-methoxyethoxyethyl)-phenylalanyl-phenylalanyl-glycyl-leucyl-methionineamide. Boc-Glu-(γ-OEEM)-Phe-Phe-Gly-Leu-Met-$NH_2$

Synthesis of tert.butyloxycarbonyl-glutamate (γ-methoxyethoxyethyl)-dibenzyl. Boc-Glu-(γ-OEEM)OBzl.

1.0 equivalents of Boc-Glu-OBzl are dissolved in $CH_2Cl_2$ and to the solution are added in the order 1.2 equivalents of methoxy-ethoxy-ethanol, 0.1 equivalents of DMAP and 1.2 equivalents of DCC.

After having verified the disappearing of the starting product, DCU is filtered and the resulting solution, after washings with citric acid at 5%, water, sodium bicarbonate at 5%, water, is evaporated to dryness, a colourless oil being obtained. The desired product is obtained by chromatography on silica with

hexane/Et/Ac (70:30, v/v) as the elutant.

Elemental analysis for $C_{22}H_{33}O_8N$

Theoretical: C, 60.13; H, 7.51; N, 3.18

Found : C, 60.00; H, 7.50; N, 3.28.

The chromatographic analyses (tlc and hplc) do not reveal presence of impurities, $^1H$ N.M.R. spectrum confirms the identity of the product.

Synthesis of tert.butyloxycarbonyl-glutamyl (γ-methoxyethoxytheyl)-phenylalanyl-phenylalanyl-glycyl-leucyl-methionineamide

Boc-Glu-(γ-OEEM)-Phe-Phe-Gly-Leu-Met-$NH_2$

1.0 Equivalents of Boc-Glu(γ-OEEM)-OH (obtained by means of the removal of benzyl ester from Boc-Glu(γ-OEEM)-OBzl by catalytic hydrogenation with Pd at 10% on carbon) are dissolved in anhydrous THF and to the solution, cooled at -15°C and kept under nitrogen atmosphere, 1.1 equivalents are added of NMM, as well as 1.1 equivalents of isobutylchloroformate. After 2 minutes, 1.0 equivalents of HCl.H-Phe-Phe-Gly-Leu-Met-$NH_2$ (obtained by removing the tert.butyloxycarbonyl group from Boc-Phe-Phe-Gly-Leu-Met-$NH_2$ with 4.5N HCl in EtAc)and 1.0 equivalents of NMM in DMF are added.

During the additions, the temperature is controlled not to increase over -10°C.

After having verified the disappearing of HCl.H-Phe-Phe-Gly-Leu-Met-$NH_2$, the reaction is stopped by evaporating the solvent mixture to dryness: to the residue, dissolved with EtAc, an excess of water is added, a white precipitate being obtained. The desired product is obtained by reverse phase chromatography using a LiChroprep RP-18 25-40 µm resin (Merck) and acetonitrile/water (22/88, v/v) as the elutant.

Melting point = 225-230

$[\alpha]_D^{22}$ = -27.3 (c = 1 in DMF).

Elemental analysis for $C_{46}H_{68}N_7O_{12}S$

Theoretical = C, 58.58; H, 7.28; N, 10.40

Found = C, 58.66; H, 7.20; N, 10.35

The analysis of the aminoacids and the $^1H$ N.M.R. spectrum confirm the identity of the product; the chromatographic analysis (tlc and hplc) does not show presence of impurities.

## Example 3

Synthesis of Arginyl-prolyl-lisy[C$N^{\epsilon}$-methoxy(ethoxy)$_2$ethyloxycarbonyl]-prolyl-glutaminyl-glutamyl(γ-methoxyethoxyethyl)-phenylalanyl-phenylalanyl-glycyl-leucyl-methionineamide. H-Arg-Pro-Lys-($N^{\epsilon}$MEEEOC)-Pro-Gln-Glu(γ-OEEM),Phe-Phe-Gly-Leu-Met-$NH_2$.

Synthesis of methoxyethoxyethyl 9-fluorenylmethoxycarbonylglutamate. Fmoc-Glu-(γ-OEEM)-OH

1.0 Equivalents of Boc-Glu(γ-OEEM)OBzl are dissolved in MeOH and treated with 10 equivalents of ammonium formate in the presence of palladium sponge (50 mg of palladium sponge per each 500 mg of Boc-Glu-(γ-OEEM)OBzl treated).

After 20 minutes the solution is filtered, and the filtrate is evaporated to dryness; the residue is dissolved with water-dioxane (1:1, v/v) and freeze-dried. The freeze-dried product is treated with a solution of TFA/ $CH_2Cl_2$ (1:1, v/v) over 30 minutes; the solvents are then evaporated to dryness; the residue is dissolved with water/ acetone (1:1, v/v), and to the solution 1.2 equivalents of Fmoc-OSu dissolved in acetone and 1.2 equivalents of sodium bicarbonate dissolved in water are then added.

After one night, from the solution acetone is evaporated off, the resulting aqueous solution is washed with $Et_2O$, made acid with HCl, and extracted a plurality of times with EtAc. The organic portions are

combined and dried over $MgSO_4$, filtered and evaporated to dryness.

The residual oil is submitted to normal-phase chromatography on Lobar Si 60 (Merck), elutant chloroform-MeOH (97:3, v/v); the desired peak is collected, evaporated to dryness and submitted again to reverse-phase chromatography on a LiChroprep RP-18 25-40 µm resin (Merck), using 0.01M ammonium acetate /$CH_3CN$ 17% as the elutant.

The desired peak is collected, acetonitrile is evaporated off and the product is recovered as an oil after freeze-drying.

Elemental analysis for $C_{25}H_{29}NO_8$

Theoretical: C, 52.72; H, 5.13; N, 2.46

Found : C, 52.65; H, 5.10; N, 2.44.

The chromatographic analyses (tlc and hplc) do not show presence of impurities, the $^1H$ N.M.R. spectrum confirms the identity of the product.

Synthesis of Benzyloxycarbonyl[Nᵋ-methoxy(ethoxy)$_2$ethyloxycarbonyl)-lysine.Z-Lys(Nᵋ-MEEEOC).OH.

1.0 Equivalents of Z-Lys-OH are dissolved in a solution of NaOH containing 1.0 equivalents of sodium hydroxide. To this solution, cooled at 5°C, 1.1 equivalents of methoxy(ethoxy)$_2$ethylchlorocarbonate dissolved in a 4.5N NaOH solution containing 1.2 equivalents of sodium hydroxide are slowly added.

After having verified the disappearing of starting Z-Lys-OH, the solution is washed twice with $Et_2O$, made acid to Congo red with a 5N HCl solution, and finally extracted many times with EtAc. The organic fractions are combined, washed with water, dried over $MgSO_4$, evaporated to dryness, a colourless oil being obtained.

Elemental analysis for $C_{22}H_{34}NO_9$

Theoretical: C, 57.88; H, 7.51; N, 3.07

Found : C, 57.73; H, 7.48; N, 3.04.

The chromatographic analyses (tlc and hplc) do not show presence of impurities, the $^1N$ N.M.R. spectrum confirms the identity of the product.

Synthesis of Fluorenylmethyloxycarbonyl[Nᵋ-methoxy(ethoxy)$_2$ethyloxycarbonyl)-lysine. Fmoc-Lys(Nᵋ-MEEEOC)-OH.

1.0 Equivalents of Z-Lys(Nᵋ-MEEEOC)-OH are dissolved in MeOH, and treated with Pd at 10% on carbon, under hydrogen bubbling.

After one houre the hydrogen flow is discontinued, the reaction mixture is filtered and evaporated to dryness.

The residue is dissolved again with a water-acetone (1:1, v/v) solution, and to the solution 1.1 equivalents of Fmoc-OSu dissolved in acetone, and 1.1 equivalents of sodium bicarbonate dissoled in water are added.

After one night, from the solution acetone is evaporated, the aqueous solution is washed with $Et_2O$, made acid to Congo red with HCl, and extracted many times with EtAc. The organic fractions are combined and dried over $MgSO_4$, filtered and evaporated to dryness.

The residual oil is submitted to reverse-phase chromatography on LiChroprep RP-18, 25-40µm resin (Merck), using 0.01M ammonium acetate/MeOH 40% as the elutant. After that the desired peak has been collected, and methanol evaporated off, the desired product is obtained by freeze-drying.

Elemental analysis for $C_{29}H_{37}NO_9$

Theoretical: C, 64.07; H, 6.86; N, 2.58;

Found : C, 63.98; H, 6.80; N, 2.55.

The $^1$H N.M.R. spectrum confirms the identity of the product; the chromatographic analysis (tlc and hplc) does not reveal presence of impurities.

Synthesis of arginyl-prolyl-lysyl[N$^{\epsilon}$-methoxy(ethoxy)$_2$ethyloxycarbonyl]-prolyl-glutaminyl-glutamyl($\gamma$-methoxyethoxyethyl)-phenylalanyl-phenylalanyl-glycyl-leucyl-methionineamide. H-Arg-Pro-Lys(N$^{\epsilon}$-MEEEOC)-Pro-Gln-Glu-($\gamma$-OEEM)-Phe-Phe-Gly-Leu-Met-$NH_2$

The synthesis is carried out on a Beckman synthesizer (Model 990) suitably modified, according to the "Fmoc-polyamide" technology as described by Atherton et al. (E. Atherton, C.J. Logan, and R.C. Sheppard, J. Chem. Soc. Perkin Trans. 1, 538, 1981), and as hereinunder summarized:

The polyamide resin (1.0 meq of sarcosine per g) is activated by treatment with ethylenediamine (16 h); a series is then carried out of standard cycles acylation-washings-unblocking of the N-terminal aminoacid protecting group using in sequence $(Fmoc\text{-}Nle)_2O$,

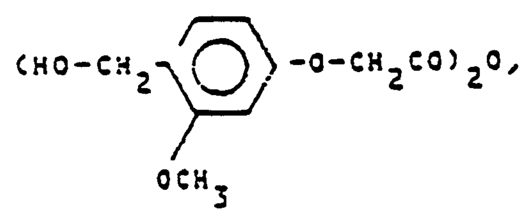

$(Fmoc\text{-}Met)_2O$ (with 1 eq. of NMM and 0.1 eq. of DMAP), $(Fmoc\text{-}Leu)_2O$, $(Fmoc\text{-}Gly)_2O$, $(Fmoc\text{-}Phe)_2O$, $(Fmoc\text{-}Phe)_2O$, Fmoc-Glu(OEEM)OH (with 1 eq. of DCC and 1 eq. of HOBt, 16 h), Fmoc-Gln-ONp (with 1 eq. of HOBt), $(Fmoc\text{-}Pro)_2O$, Fmoc-Lys(N-Meeeoc)OH with 1 eq. of DCC and 1 eq. of HOBt, 16 h, the unblocking of the undecapeptide protected by the resin is carried out with TFA 1% in $CH_2Cl_2$ containing 5% of MeSEt as the scavenger, 3h. The reaction solvents are evaporated off to dryness, and the residue is redissolved with DMF.

To the solution of N$^{\alpha}$-Fmoc-Arg$^1$(N$^G$Mtr), Lys$^3$(N$^{\epsilon}$-MEEEOC), Glu$^6$($\gamma$-OEEM -SP-OH in DMF/$CH_2Cl_2$ - (1:1), 1.1 equivalents of ammonium salt of HOBt (dissolved in DMF) and 1.1 eq. of DCC are added, and the mixture is allowed to react over 16 hours, the disappearing of the starting compound being monitored via hplc.

At the end of the reaction, the solvents are evaporated off, and a piperidine/DMF (20:80) solution is added, allowing the mixture to react over 30 minutes.

The solvent is evaporated off, and a solution of TFA-Phenol-MeSEt (95:2.5:2.5) is added, allowing the mixture to react over 6 h. After freeze-drying, the undecapeptideamide is purified sequentially by ion-exchange chromatography (resin, Whatman CM-52, linear gradient 0.01M-0.01M of $NH_4OAc$).

The purified peptide results homogeneous on hplc, tlc chromatography, and shows the correct analysis of aminoacid content: Arg, 0.90; Lys, 0.95; Phe, 1.80; Leu, 1.00; Met, 1.01; Pro, 2.04; Gly, 0.99; Glu, 2.19).

## Claims

**Claims for the following Contracting States: BE, CH, DE, FR, GB, LI, LU, NL, SE**

**1.** Analogs of "Substance P" represented by the general formula:

$R\text{-}R^5\text{-}R^4\text{-}R^3\text{-}R^2\text{-}R^1\text{-}NH_2$ (I)

at least one amino acid of which bears, chemically bonded, in the case of an acidic amino acid, a methoxy(ethoxy)$_n$ethoxy substituent of general formula:

$CH_3\text{-}O\text{-}(CH_2\text{-}CH_2\text{-}O)_n\text{-}CH_2\text{-}CH_2\text{-}O\text{-}$ (II) (MEE)

with $n$ = 0, 1, 2, 3,
and/or, in the case of a basic amino acid, a methoxy(ethoxy)$_n$ethoxycarbonyl substituent of the general formula:

$CH_3$-O-($CH_2$-$CH_2$-O-)$_n$-$CH_2$-$CH_2$-O-CO- (III) (MEEEOC)

with $n$ = 0, 1, 2, 3,
wherein:

$R^1$ is a residue of methionine, methionine sulphoxide, methionine sulphone, or seleno-methionine;
$R^2$ a residue of leucine, norleucine, or isoleucine;
$R^3$ is a residue of glycine;
$R^4$ is a residue of phenylalanine, tryptophan, or tyrosine;
$R^5$ is a residue of phenylalanine, tyrosine, 4-chlorophenylalanine, o-benzyltyrosine, and acetyl-, cyclopentyl-, tert.butyloxycarbonyl- and 4-hydroxyphenylacetyl derivatives thereof, and R is selected from among:

a) a group having the general formula (III) (MEEEOC) wherein $n$ = 0, 1, 2, 3;
b) a derivative of an amino acid having the general formula:

P-G (IV)

wherein G is a residue having the general formula:

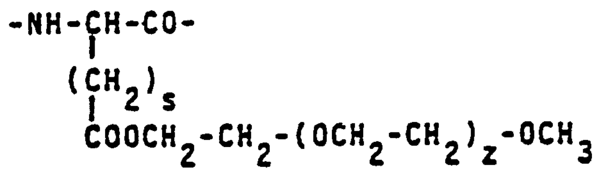
(V)

in which $s$ is 1 or 2, and $z$ is 0, 1, 2, 3, and P is a hydrogen atom, a linear or a branched aliphatic alkyl group having from 1 to 6 carbon atoms, or a linear or a branched, saturated- or unsaturated-chain aliphatic acyl group selected from among formyl, acetyl, propionyl, n-butyryl, isobutyryl, n-valeryl, isovaleryl, hexanoyl, isohexanoyl, heptanoyl, octanoyl, crotonoyl, methacryloyl, acryloyl, or a substituted acyl group selected from among: hydroxyacetyl, 2-hydroxypropanoyl, 2-hydroxypropanoyl, 3-hydroxypropanoyl, aminoacetyl, 4-hydroxyphenylacetyl, 4-hydroxyphenylpropanoyl, 2-aminopropanoyl, 3-aminopropanoyl, o-ethylmalonyl, ethoxyformyl, methoxyformyl, 3-methoxypropanoyl, 3-ethoxypropanoyl, chloroacetyl, dichloroacetyl, 2-chloropropanoyl, 3-chloropropanoyl, 2,3-dichloropropanoyl, bromoacetyl, 4-hydroxy-3,5-diiodophenylacetyl, 3-oxobutyryl, 3-oxovaleryl, methylthioacetyl, 3-methylthiopropanoyl, ethylthioacetyl, 3-ethylthiopropanoyl, nicotinoyl, 4-aminobutyryl, $N^{\alpha}$-[1-(9-adenyl)-$\beta$-D-ribofuranuronosyl)], $N^{\alpha}$-[(1-(9-hypoxanthyl)-$\beta$-D-ribofuranuronosyl)], or a group selected from among benzyloxycarbonyl, tert.butyloxycarbonyl, tert.amiloxycarbonyl, isobornyloxycarbonyl, adamantyloxycarbonyl, chloro- or nitro substituted benzyloxycarbonyl;
c) an amino acid derivative having the general formula:

$CH_3$-O($CH_2$-$CH_2$-O)$_n$-$CH_2$-$CH_2$-O-CO-$R^6$ (VI)

wherein n is 0, 1, 2, 3, and $R^6$ is a residue of glutamine and the MEEEOC derivative thereof, or a residue of formula (V) wherein $s$ and $z$ are as defined above;
d) a derivative of a dipeptidic segment having the general formula:

$CH_3$-O-($CH_2$-$CH_2$-O)$_n$-$CH_2$-$CH_2$-O-CO-$R^8$-$R^7$ (VII)

wherein n is 0, 1, 2, 3, $R^7$ is a residue of glutamine or a residue of formula (V), wherein $s$ and $z$ are as defined above, and $R^8$ is a residue of glutamine or the MEEEOC derivative thereof, or a residue having the general formula:

$$-NH-CH-CO-$$
$$(CH_2)_h$$
$$COO-CH_2-CH_2-(OCH_2-CH_2)_k-O-CH_3 \qquad (VIII)$$

wherein h is 1 or 2, and k is 0, 1, 2, 3;

e) a derivative of a dipeptidic segment having the general formula:

$P-R^{10}-R^9$ (IX)

wherein P has the previously disclosed meaning, $R^9$ is a residue of formula (V), and $R^{10}$ is a residue of glutamine or the MEEEOC derivative thereof, or a derivative of formula (VIII), or wherein $R^{10}$ is a residue of formula (V) and $R^9$ is a residue of glutamine or the MEEEOC derivative thereof, or a residue of formula (VIII);

f) a derivative of a tripeptidic segment having the general formula:

$CH_3-O-(CH_2-CH_2-O)_n-CH_2-CH_2-O-CO-R^{11}-R^8-R^7$ (X)

wherein n, $R^8$ and $R^7$ have the previously indicated meanings, and $R^{11}$ is a residue of proline;

g) a derivative of a tripeptidic segment having the general formula:

$P-R^{11}-R^9-R^{10}$ (XI)

wherein P, $R^9$, $R^{10}$ and $R^{11}$ have the previously disclosed meanings;

h) a tetrapeptidic derivative having the general formula:

$CH_3-O(CH_2-CH_2-O-)_n-CH_2-CH_2-O-CO-R^{12}-R^{11}-R^8-R^7$ (XII)

wherein n, $R^7$, $R^8$, and $R^{11}$ have the previously disclosed meanings, and $R^{12}$ is a residue of lysine or the MEEEOC derivative thereof, or is a residue having the general formula:

$$-NH-CH-CO-$$
$$(CH_2)_j$$
$$NH-CO-O-CH_2-CH_2-(O-CH_2-CH_2)_i-OCH_3 \qquad (XIII)$$

wherein i is 0, 1, 2, 3, and j is 1, 2, 3, 4;

i) a tetrapeptidic derivative having the general formula:

$P-R^{12}-R^{11}-R^{10}-R^9$ (XIV)

wherein P, $R^9$, $R^{10}$, $R^{11}$ and $R^{12}$ have the previously disclosed meanings;

j) a pentapeptide derivative having the general formula:

$CH_3-O(CH_2-CH_2-O-)_n-CH_2-CH_2-O-CO-R^{13}-R^{12}-R^{11}-R^8-R^7$ (XV)

wherein n, $R^7$, $R^8$, $R^{11}$, and $R^{12}$ have the previously disclosed meanings, and $R^{13}$ is a residue of proline;

k) a pentapeptide derivative having the general formula:

$P-R^{13}-R^{12}-R^{11}-R^{10}-R^9$ (XVI)

wherein P, $R^9$, $R^{10}$, $R^{11}$, $R^{12}$ and $R^{13}$ have the previously disclosed meanings;

l) a hexapeptide derivative having the general formula:

$CH_3$-O($CH_2$-$CH_2$-O)$_n$-$CH_2$-$CH_2$-O-CO-$R^{14}$-$R^{13}$-$R^{12}$-$R^{11}$-$R^8$-$R^7$ (XVII)

wherein n, $R^7$, $R^8$, $R^{11}$, $R^{12}$ and $R^{13}$ have the previously disclosed meanings, and $R^{14}$ is a residue of arginine and the MEEEOC derivative thereof, or

m) a hexapeptidic derivative having the general formula:

P-$R^{14}$-$R^{13}$-$R^{12}$-$R^{11}$-$R^{10}$-$R^9$ (XVIII)

wherein P, $R^9$, $R^{10}$, $R^{11}$, $R^{12}$, $R^{13}$ and $R^{14}$ have the previously disclosed meanings.

**2.** Analogs of Substance P according to claim 1 wherein:
R is selected from among:

a) a group of formula (III) (MEEEOC);

b) an amino acid derivative of formula (IV) wherein G is a residue of formula (V), and P, s, and z have the previously disclosed meanings;

c) an amino acid derivative of formula (VI), wherein n and $R^6$ have the previously disclosed meanings, or a residue of formula (V), wherein s and z have the previously disclosed meanings;

d) a derivative of a dipeptidic segment of formula (VII), wherein n has the previously disclosed meaning and $R^7$ is a residue of glutamine, or a residue of formula (V) wherein s and z have the previously disclosed meanings and $R^8$ is a residue of glutamine or the MEEEOC derivative thereof, or a residue of formula (VIII) wherein h and k have the previously disclosed meanings;

e) a derivative of a dipeptidic segment of formula (IX), wherein P has the previously disclosed meaning and $R^9$ is a residue of formula (V) wherein s and z have the previously disclosed meanings, and $R^{10}$ is a residue of glutamine or the MEEEOC derivative thereof, or a derivative of formula (VIII) wherein h and k have the previously disclosed meanings, or $R^{10}$ is a residue of formula (V) wherein s and z have the previously disclosed meanings, and $R^9$ is a residue of glutamine or the MEEEOC derivative thereof, or a residue of formula (VIII) wherein h and k have the previously disclosed meanings;

f) a derivative of a tripeptidic segment of formula (X) wherein n, $R^7$, $R^8$ and $R^{11}$ have the previously disclosed meanings;

g) a derivative of a tripeptidic segment of formula (XI) wherein P, $R^9$, $R^{10}$ and $R^{11}$ have the previsouly disclosed meanings;

h) a hexapeptidic derivative of formula (XVII) wherein n, $R^7$, $R^8$, $R^{11}$, $R^{13}$ and $R^{14}$ have the previously disclosed meanings, and

i) a hexapeptidic segment of formula (XVIII) wherein P, $R^9$, $R^{10}$, $R^{11}$, $R^{12}$, $R^{13}$ and $R^{14}$ have the previously disclosed meanings.

**3.** Peptide according to claim 1, having the formula:
MEEEOC-Phe-Phe-Gly-Leu-Met-$NH_2$
wherein MEEEOC is $CH_3$-O($CH_2$-$CH_2$-O)$_2$-$CH_2$-$CH_2$-OCO-, and all the amino acids have an L-configuration.

**4.** Peptide according to claim 1, having the formula:
MEEEOC-Gln-Phe-Phe-Gly-Leu-Met-$NH_2$
wherein MEEEOC has the previously disclosed meaning and all the amino acids have an L-configuration.

**5.** Peptide according to claim 1, having the formula:
MEEEOC-Gln-Gln-Phe-Phe-Gly-Leu-Met-$NH_2$
wherein MEEEOC has the previously disclosed meaning and all the amino acids have an L-configuration.

**6.** The peptide having the formula:
MEEEOC-Pro-Gln-Phe-Phe-Gly-Leu-Met-$NH_2$
wherein MEEEOC has the previously disclosed meaning and all the amino acids have an L-configuration.

7. Peptide according to claim 1, having the formula:
MEEEOC-Glu($\gamma$-OEEM)-Phe-Phe-Gly-Leu-Met-$NH_2$
wherein MEEEOC has the previously disclosed meaning, OEEM is $CH_3$-$CH_2$-$CH_2$-O-$CH_2$-$CH_2$-O- and all the amino acids have an L-configuration.

8. Peptide according to claim 1, having the formula:
MEEEOC-Gln-Glu-($\gamma$-OEEM)-Phe-Phe-Gly-Leu-Met-$NH_2$
wherein MEEEOC and OEEM have the previously disclosed meanings and all the amino acids have an L-configuration.

9. Peptide according to claim 1, having the formula:
MEEEOC-Pro-Gln-Glu-($\gamma$-OEEM)-Phe-Phe-Gly-Leu-Met-$NH_2$
wherein MEEEOC and OEEM have the previously disclosed meanings and all the amino acids have an L-configuration.

10. Peptide according to claim 1, having the formula:
MEEEOC-Glu-($\gamma$-OEEM)-Gln-Phe-Phe-Gly-Leu-Met-$NH_2$
wherein MEEEOC and OEEM have the previously disclosed meanings and all the amino acids have an L-configuration.

11. Peptide according to claim 1, having the formula:
MEEEOC-Pro-Glu-($\gamma$-OEEM)-Gln-Phe-Phe-Gly-Leu-Met-$NH_2$
wherein MEEEOC and OEEM have the previously disclosed meanings and all the amino acids have an L-configuration.

12. Peptide according to claim 1, having the formula:
MEEEOC-Glu-($\gamma$-OEEM)-Glu ($\gamma$-OEEM)-Phe-Phe-Gly-Leu-Met-$NH_2$
wherein MEEEOC and OEEM have the previously disclosed meanings and all the amino acids have an L-configuration.

13. Peptide according to claim 1, having the formula:
MEEEOC-Pro-Glu-($\gamma$-OEEM)-Glu-($\gamma$-OEEM)-Phe-Phe-Gly-Leu-Met-$NH_2$
wherein MEEEOC and OEEM have the previously disclosed meanings and all the aminoacids have an L-configuration.

14. Peptide according to claim 1, having the formula:
MEEEOC-Arg-Pro-Lys-Pro-Gln-Gln-Phe-Phe-Gly-Leu-Met-$NH_2$
wherein MEEEOC and OEEM have the previously disclosed meanings and all the amino acids have an L-configuration.

15. Peptide according to claim 1, having the formula:
MEEEOC-Arg-Pro-Lys-Pro-Glu-($\gamma$-OEEM)-Gln-Phe-Phe-Gly-Leu--Met-$NH_2$
wherein MEEEOC and OEEM have the previously disclosed meanings and all the amino acids have an L-configuration.

16. Peptide according to claim 1, having the formula:
MEEEOC-Arg-Pro-Lys-Pro-Gln-Glu-($\gamma$-OEEM)-Phe-Phe-Gly-Leu-Met-$NH_2$
wherein MEEEOC and OEEM have the previously disclosed meanings and all the amino acids have an L-configuration.

17. Peptide according to claim 1, having the formula:
H-Arg-Pro-Lys-($N^{\epsilon}$-MEEEOC)-Pro-Gln-Gln-Phe-Phe-Gly-Leu-Met-$NH_2$
wherein MEEEOC has the previously disclosed meaning and all the amino acids have an L-configuration.

18. Peptide according to claim 1, having the formula:

H-Arg-Pro-Lys-($N^{\epsilon}$-MEEEOC)-Pro-Gln-Glu-($\gamma$-OEEM)-Phe-Phe-Gly-Leu-Met-$NH_2$
wherein MEEEOC and OEEM have the previously disclosed meanings and all the amino acids have an L-configuration.

**19.** Peptide according to claim 1, having the formula:
H-Arg-Pro-Lys-($N^{\epsilon}$-MEEEOC)-Pro-Glu-($\gamma$-OEEM)-Gln-Phe-Phe-Gly-Leu-Met-$NH_2$
wherein MEEEOC and OEEM have the previously disclosed meanings and all the amino acids have an L-configuration.

**20.** Peptide according to claim 1, having the formula:
H-Arg-Pro-Lys-($N^{\epsilon}$-MEEEOC)-Pro-Glu-($\gamma$-OEEM)-Glu-($\gamma$-OEEM)-Phe-Phe-Gly-Leu-Met-$NH_2$
wherein MEEEOC and OEEM have the previously disclosed meanings and all the amino acids have an L-configuration.

**Claims for the following Contracting State: AT**

**1.** Process for preparing analogs of Substance P represented by the general formula:

$R-R^5-R^4-R^3-R^2-R^1-NH_2$ (I)

at least one amino acid of which bears, chemically bonded, in the case of an acidic amino acid, a methoxy(ethoxy)$_n$ethoxy substituent of general formula:

$CH_3-O-(CH_2-CH_2-O)_n-CH_2-CH_2-O-$ (II) (MEE)

with $\underline{n}$ = 0, 1, 2, 3,
and/or, in the case of a basic amino acid, a methoxy(ethoxy)$_n$ethoxycarbonyl substituent of the general formula:

$CH_3-O-(CH_2-CH_2-O-)_n-CH_2-CH_2-O-CO-$ (III) (MEEEOC)

with $\underline{n}$ = 0, 1, 2, 3,
wherein:
$R^1$ is a residue of methionine, methionine sulphoxide, methionine sulphone, or seleno-methionine;
$R^2$ a residue of leucine, norleucine, or isoleucine;
$R^3$ is a residue of glycine;
$R^4$ is a residue of phenylalanine, tryptophan, or tyrosine;
$R^5$ is a residue of phenylalanine, tyrosine, 4-chlorophenylalanine, o-benzyltyrosine, and acetyl-, cyclopentyl-, tert.butyloxycarbonyl- and 4-hydroxyphenylacetyl derivatives thereof, and R is selected from among:
a) a group having the general formula (III) (MEEEOC) wherein $\underline{n}$ = 0, 1, 2, 3;
b) a derivative of an amino acid having the general formula:

P-G (IV)

wherein G is a residue having the general formula:

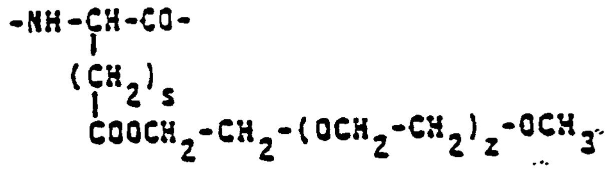 (V)

in which $\underline{s}$ is 1 or 2, and $\underline{z}$ is 0, 1, 2, 3, and P is a hydrogen atom, a linear or a branched aliphatic alkyl group having from 1 to 6 carbon atoms, or a linear or a branched, saturated- or unsaturated-chain aliphatic acyl group selected from among formyl, acetyl, propionyl, n-butyryl, isobutyryl, n-valeryl, isovaleryl, hexanoyl, isohexanoyl, heptanoyl, octanoyl, crotonoyl, methacryloyl, acryloyl, or

a substituted acyl group selected from among: hydroxyacetyl, 2-hydroxypropanoyl, 2-hydroxypropanoyl, 3-hydroxypropanoyl, aminoacetyl, 4-hydroxyphenylacetyl, 4-hydroxyphenylpropanoyl, 2-aminopropanoyl, 3-aminopropanoyl, o-ethylmalonyl, ethoxyformyl, methoxyformyl, 3-methoxypropanoyl, 3-ethoxypropanoyl, chloroacetyl, dichloroacetyl, 2-chloropropanoyl, 3-chloropropanoyl, 2,3-dichloropropanoyl, bromoacetyl, 4-hydroxy-3,5-diiodophenylacetyl, 3-oxobutyryl, 3-oxovaleryl, methylthioacetyl, 3-methylthiopropanoyl, ethylthioacetyl, 3-ethylthiopropanoyl, nicotinoyl, 4-aminobutyryl, $N^{\alpha}$-[1-(9-adenyl)-$\beta$-D-ribofuranuronosyl)], $N^{\alpha}$-[(1-(9-hypoxanthyl)-$\beta$-D-ribofuranuronosyl)], or a group selected from among benzyloxycarbonyl, tert.butyloxycarbonyl, tert.amiloxycarbonyl, isobornyloxycarbonyl, adamantyloxycarbonyl, chloro- or nitro substituted benzyloxycarbonyl;

c) an amino acid derivative having the general formula:

$CH_3$-O($CH_2$-$CH_2$-O)$_n$-$CH_2$-$CH_2$-O-CO-$R^6$ (VI)

wherein n is 0, 1, 2, 3, and $R^6$ is a residue of glutamine and the MEEEOC derivative thereof, or a residue of formula (V) wherein s and z are as defined above;

d) a derivative of a dipeptidic segment having the general formula:

$CH_3$-O-($CH_2$-$CH_2$-O)$_n$-$CH_2$-$CH_2$-O-CO-$R^8$-$R^7$ (VII)

wherein n is 0, 1, 2, 3, $R^7$ is a residue of glutamine or a residue of formula (V), wherein s and z are as defined above, and $R^8$ is a residue of glutamine or the MEEEOC derivative thereof, or a residue having the general formula:

-NH-CH-CO-
(CH$_2$)$_h$
COO-$CH_2$-$CH_2$-(O$CH_2$-$CH_2$)$_k$-O-$CH_3$ (VIII)

wherein h is 1 or 2, and k is 0, 1, 2, 3;

e) a derivative of a dipeptidic segment having the general formula:

P-$R^{10}$-$R^9$ (IX)

wherein P has the previously disclosed meaning, $R^9$ is a residue of formula (V), and $R^{10}$ is a residue of glutamine or the MEEEOC derivative thereof, or a derivative of formula (VIII), or wherein $R^{10}$ is a residue of formula (V) and $R^9$ is a residue of glutamine or the MEEEOC derivative thereof, or a residue of formula (VIII);

f) a derivative of a tripeptidic segment having the general formula:

$CH_3$-O-($CH_2$-$CH_2$-O)$_n$-$CH_2$-$CH_2$-O-CO-$R^{11}$-$R^8$-$R^7$ (X)

wherein n, $R^8$ and $R^7$ have the previously indicated meanings, and $R^{11}$ is a residue of proline;

g) a derivative of a tripeptidic segment having the general formula:

P-$R^{11}$-$R^9$-$R^{10}$ (XI)

wherein P, $R^9$, $R^{10}$ and $R^{11}$ have the previously disclosed meanings;

h) a tetrapeptidic derivative having the general formula:

$CH_3$-O($CH_2$-$CH_2$-O-)$_n$-$CH_2$-$CH_2$-O-CO-$R^{12}$-$R^{11}$-$R^8$-$R^7$ (XII)

wherein n, $R^7$, $R^8$, and $R^{11}$ have the previously disclosed meanings, and $R^{12}$ is a residue of lysine or the MEEEOC derivative thereof, or is a residue having the general formula:

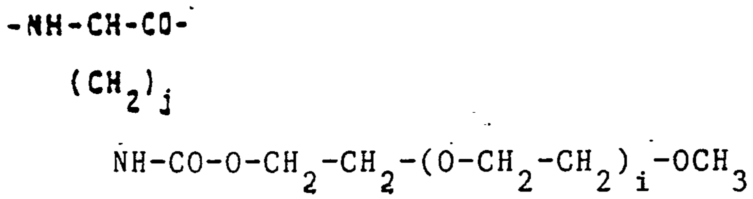
(XIII)

wherein i is 0, 1, 2, 3, and j is 1, 2, 3, 4;

i) a tetrapeptidic derivative having the general formula:

$P-R^{12}-R^{11}-R^{10}-R^{9}$ (XIV)

wherein P, $R^9$, $R^{10}$, $R^{11}$ and $R^{12}$ have the previously disclosed meanings;

j) a pentapeptide derivative having the general formula:

$CH_3-O(CH_2-CH_2-O-)_n-CH_2-CH_2-O-CO-R^{13}-R^{12}-R^{11}-R^{8}-R^{7}$ (XV)

wherein n, $R^7$, $R^8$, $R^{11}$, and $R^{12}$ have the previously disclosed meanings, and $R^{13}$ is a residue of proline;

k) a pentapeptide derivative having the general formula:

$P-R^{13}-R^{12}-R^{11}-R^{10}-R^{9}$ (XVI)

wherein P, $R^9$, $R^{10}$, $R^{11}$, $R^{12}$ and $R^{13}$ have the previously disclosed meanings;

l) a hexapeptide derivative having the general formula:

$CH_3-O(CH_2-CH_2-O)_n-CH_2-CH_2-O-CO-R^{14}-R^{13}-R^{12}-R^{11}-R^{8}-R^{7}$ (XVII)

wherein n, $R^7$, $R^8$, $R^{11}$, $R^{12}$ and $R^{13}$ have the previously disclosed meanings, and $R^{14}$ is a residue of arginine and the MEEEOC derivative thereof, or

m) a hexapeptidic derivative having the general formula

$P-R^{14}-R^{13}-R^{12}-R^{11}-R^{10}-R^{9}$ (XVIII)

wherein P, $R^9$, $R^{10}$, $R^{11}$, $R^{12}$,$R^{13}$ and $R^{14}$ have the previously disclosed meanings,

characterized in that the groups represented by the formula (II) are introduced into the molecule of Substance P as esters or residues of glutamine or aspartic acid, whereas the groups represented by the general formula (III) are introduced into the molecule of Substance P by means of the reaction of methoxy(ethoxy)$_n$ethylchlorocarbonate, with formula:

$CH_3-O(CH_2-CH_2-O)_n$ $CH_2-CH_2-O-CO-Cl$

with the selected aminoacid, after having suitably protected all other reactive functional groups present.

**2.** Process according to claim 1, characterized in that the esters of glutamic or aspartic acid are prepared with derivatives of the aminoacid concerned, protected at both the $-NH_2$ and the -COOH groups by causing the free carboxy groups to condense with methoxy(ethoxy)$_n$ ethanol in the presence of N,N-dicyclohexylcarbodiimide and N,N-dimethylaminopyridine.

**3.** Process according to claim 1, characterized in that the aminoacid derivatives which contain the groups represented by the formulae (II) and (III) are incorporated into a polypeptidic chain by condensation with N,N-dicyclohexylcarbodiimide/hydroxybenzotriazole and activation of -COOH by mixed anhydrided, or symmetrical anhydrides, or active esters.

**Revendications**

**Revendications pour les Etats contractants suivants: BE, CH, DE, FR, GB, LI, LU, NL, SE**

1. Analogues de la "substance P", représentés par la formule générale :

$R\text{-}R^5\text{-}R^4\text{-}R^3\text{-}R^2\text{-}R^1\text{-}NH_2$ (I)

dont au moins un acide aminé porte, chimiquement lié, dans le cas d'un acide aminé acide, un substituant méthoxy(éthoxy)$_n$-éthoxy, de formule générale :

$CH_3\text{-}O\text{-}(CH_2\text{-}CH_2\text{-}O)_n\text{-}CH_2\text{-}CH_2\text{-}O\text{-}$ (II)(MEE)

avec $\underline{n}$ = 0, 1, 2, 3,
et/ou, dans le cas d'un acide aminé basique, un substituant méthoxy-(éthoxy)$_n$-éthoxycarbonyle de formule générale :

$CH_3\text{-}O\text{-}(CH_2\text{-}CH_2\text{-}O)_n\text{-}CH_2\text{-}CH_2\text{-}O\text{-}CO\text{-}$ (III)(MEEEOC)

avec $\underline{n}$ = 0, 1, 2, 3,
dans laquelle :

$R^1$ est un résidu de méthionine, de méthionine-sulfoxyde, de méthionine-sulfone, ou de sélénométhionine,
$R^2$ est un résidu de leucine, de norleucine ou d'isoleucine,
$R^3$ est un résidu de glycine,
$R^4$ est un résidu de phénylalanine, de tryptophane ou de tyrosine,
$R^5$ est un résidu de phénylalanine, de tyrosine, de 4-chloro-phénylalanine, d'o-benzyltyrosine, ou de leurs dérivés à substituant acétyle, cyclopentyle, t-butyloxycarbonyle ou 4-hydroxyphényl-acétyle, et
R est choisi parmi :

a) un groupe présentant la formule générale (III) (MEEEOC) dans laquelle $\underline{n}$ vaut 0, 1, 2 ou 3 ;
b) un dérivé d'acide aminé, présentant la formule générale :

P-G (IV)

dans laquelle G est un résidu présentant la formule générale :

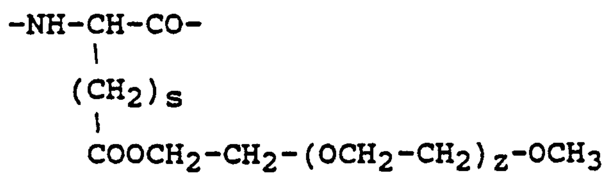 (V)

dans laquelle $\underline{s}$ vaut 1 ou 2 et $\underline{z}$ vaut 0, 1, 2 ou 3, et P représente un atome d'hydrogène, un groupe alkyle aliphatique linéaire ou ramifié, comportant de 1 à 6 atomes de carbone, ou un groupe acyle aliphatique linéaire ou ramifié, saturé ou non, choisi parmi formyle, acétyle, propionyle, n-butyryle, isobutyryle, n-valéryle, isovaléryle, hexanoyle, isohexanoyle, heptanoyle, octanoyle, crotonoyle, méthacryloyle, acryloyle, ou encore un groupe acyle substitué, choisi parmi hydroxyacétyle, 2-hydroxypropanoyle, 2-hydroxypropanoyle, 3-hydroxypropanoyle, aminoacétyle, 4-hydroxyphénylacétyle, 4-hydroxyphénylpropanoyle, 2-aminopropanoyle, 3-aminopropanoyle, o-éthylmalonyle, éthoxyformyle, méthoxyformyle, 3-méthoxypropanoyle, 3-éthoxypropanoyle, chloroacétyle, dichloro-acétyle, 2-chloropropanoyle, 3-chloropropanoyle, 2,3-dichloropropanoyle, bromoacétyle, 4-hydroxy-3,5-diiodophénylacétyle, 3-oxobutyryle, 3-oxovaléryle, méthylthioacétyle, 3-méthylthiopropanoyle, éthylthioacétyle, 3-éthylthiopropanoyle, nicotinoyle, 4-aminobutyryle, $N^\alpha$-[1-(9-adényl)-$\beta$-D-ribofuranuronosyle)], $N^\alpha$-[(1-(9-hypoxanthyl)-$\beta$-D-ribofuranuronosyle)] ou encore un groupe choisi parmi benzyloxycarbonyle, tertiobutyloxycarbonyle, tertioamyloxycarbonyle, isobornyloxycarbonyle, adamantyloxycarbonyle, benzyloxycarbonyle à substituant chloro ou nitro ;

c) un dérivé d'acide aminé présentant la formule générale :

$CH_3\text{-}O(CH_2\text{-}CH_2\text{-}O)_n\text{-}CH_2CH_2\text{-}O\text{-}CO\text{-}R^6$ (IV)

dans laquelle n vaut 0, 1, 2 ou 3 et $R^6$ représente un résidu de glutamine ou son dérivé MEEEOC, ou un résidu de formule (V) dans laquelle s et z sont tels que définis cidessus ;
d) un dérivé d'un segment dipeptidique présentant la formule générale :

$CH_3$-O-($CH_2$-$CH_2$-O$)_n$-$CH_2$-$CH_2$-O-CO-$R^8$-$R^7$ (VII)

dans laquelle n vaut 0, 1, 2 ou 3, $R^7$ représente un résidu de glutamine ou un résidu de formule (V), dans laquelle s et z sont tels que définis ci-dessus, et $R^8$ représente un résidu de glutamine ou son dérivé MEEEOC, ou encore un résidu présentant la formule générale :

```
-NH-CH-CO-
     |
    (CH2)h
     |
    COO-CH2-CH2-(OCH2-CH2)k-O-CH3   (VIII)
```

dans laquelle h vaut 1 ou 2 et k vaut 0, 1, 2 ou 3 ;
e) un dérivé d'un segment dipeptidique présentant la formule générale :

P-$R^{10}$-$R^9$ (IX)

dans laquelle P présente la signification décrite précédemment, $R^9$ représente un résidu de formule (V), et $R^{10}$ représente un résidu de glutamine ou son dérivé MEEEOC, ou encore un résidu de formule (VIII), ou bien dans laquelle $R^{10}$ représente un résidu de formule (V) et $R^9$ représente un résidu de glutamine ou son dérivé MEEEOC, ou encore un résidu de formule (VIII) ;
f) un dérivé d'un segment tripeptidique présentant la formule générale :

$CH_3$-O-($CH_2$-$CH_2$-O$)_n$-$CH_2$-$CH_2$-O-CO-$R^{11}$-$R^8$-$R^7$ (X)

dans laquelle n, $R^8$ et $R^7$ ont les significations indiquées précédemment, et $R^{11}$ représente un résidu de proline ;
g) un dérivé d'un segment tripeptidique présentant la formule générale :

P-$R^{11}$-$R^9$-$R^{10}$ (XI)

dans laquelle P, $R^9$, $R^{10}$ et $R^{11}$ ont les significations indiquées précédemment ;
h) un dérivé tétrapeptidique présentant la formule générale :

$CH_3$-O($CH_2$-$CH_2$-O$)_n$-$CH_2$-$CH_2$-O-CO-$R^{12}$-$R^{11}$-$R^8$-$R^7$ (XII)

dans laquelle n, $R^7$, $R^8$ et $R^{11}$ ont les significations indiquées précédemment, et $R^{12}$ représente un résidu de lysine ou son dérivé MEEEOC, ou encore un résidu présentant la formule générale :

```
-NH-CH-CO-
     |
    (CH2)j
     |
    NH-CO-O-CH2-CH2-(O-CH2-CH2)i-OCH3        (XIII)
```

dans laquelle i vaut 0, 1, 2 ou 3 et j vaut 1, 2, 3 ou 4 ;
i) un dérivé tétrapeptidique présentant la formule générale :

P-$R^{12}$-$R^{11}$-$R^{10}$-$R^9$ (XIV)

dans laquelle P, $R^9$, $R^{10}$, $R^{11}$ et $R^{12}$ ont les significations indiquées précédemment ;
j) un dérivé pentapeptidique présentant la formule générale :

$CH_3$-$O(CH_2$-$CH_2$-O-$)_n$-$CH_2$-$CH_2$-O-CO-$R^{13}$-$R^{12}$-$R^{11}$-$R^8$-$R^7$ (XV)

dans laquelle n, $R^7$, $R^8$, $R^{11}$ et $R^{12}$ ont les significations indiquées précédemment, et $R^{13}$ représente un résidu de proline ;
k) un dérivé pentapeptidique présentant la formule générale :

P-$R^{13}$-$R^{12}$-$R^{11}$-$R^{10}$-$R^9$ (XVI)

dans laquelle P, $R^9$, $R^{10}$, $R^{11}$, $R^{12}$ et $R^{13}$ ont les significations indiquées précédemment ;
l) un dérivé hexapeptidique présentant la formule générale :

$CH_3$-$O(CH_2$-$CH_2$-$O)_n$-$CH_2$-$CH_2$-O-CO-$R^{14}$-$R^{13}$-$R^{12}$-$R^{11}$-$R^8$-$R^7$ (XVII)

dans laquelle n, $R^7$, $R^8$, $R^{11}$, $R^{12}$ et $R^{13}$ ont les significations indiquées précédemment, et $R^{14}$ représente un résidu d'arginine ou son dérivé MEEEOC ; ou
m) un dérivé hexapeptidique présentant la formule générale :

P-$R^{14}$-$R^{13}$-$R^{12}$-$R^{11}$-$R^{10}$-$R^9$ (XVIII)

dans laquelle P, $R^9$, $R^{10}$, $R^{11}$, $R^{12}$, $R^{13}$ et $R^{14}$ ont les significations indiquées précédemment.

**2.** Analogues de la substance P conformes à la revendication 1, dans lesquels :
R est choisi parmi :
a) un groupe de formule (III) (MEEEOC) ;
b) un dérivé d'acide aminé de formule (IV) dans laquelle G représente un résidu de formule (V) et P, s et z ont les significations indiquées précédemment ;
c) un dérivé d'acide aminé de formule (VI) dans laquelle n et $R^6$ ont les significations indiquées précédemment, ou $R^6$ représente un résidu de formule (V) dans laquelle s et z ont les significations indiquées précédemment ;
d) un dérivé d'un segment dipeptidique de formule (VII), dans laquelle n a la signification indiquée précédemment et $R^7$ représente un résidu de glutamine ou un résidu de formule (V) dans laquelle s et z ont les significations indiquées précédemment et $R^8$ représente un résidu de glutamine ou son dérivé MEEEOC ou encore un résidu de formule (VIII) dans laquelle h et k ont les significations indiquées précédemment ;
e) un dérivé d'un segment dipeptidique de formule (IX), dans laquelle P a la signification indiquée précédemment et $R^9$ représente un résidu de formule (V) dans laquelle s et z ont les significations indiquées précédemment, et $R^{10}$ représente un résidu de glutamine ou son dérivé MEEEOC, ou encore un dérivé de formule (VIII) dans laquelle h et k ont les significations indiquées précédemment, ou bien $R^{10}$ représente un résidu de formule (V) dans laquelle s et z sont les significations indiquées précédemment et $R^9$ représente un résidu de glutamine ou son dérivé MEEEOC ou encore un résidu de formule (VIII) dans laquelle h et k ont les significations indiquées précédemment ;
f) un dérivé d'un segment tripeptidique de formule (X) dans laquelle n, $R^7$, $R^8$ et $R^{11}$ ont les significations indiquées précédemment ;
g) un dérivé d'un segment tripeptidique de formule (XI) dans laquelle P, $R^9$, $R^{10}$ et $R^{11}$ ont les significations indiquées précédemment ;
h) un dérivé hexapeptitique de formule (XVII) dans laquelle n, $R^7$, $R^8$, $R^{11}$, $R^{13}$ et $R^{14}$ ont les significations indiquées précédemment ; et
i) un segment hexapeptidique de formule (XVIII) dans laquelle P, $R^9$, $R^{10}$, $R^{11}$, $R^{12}$, $R^{13}$ et $R^{14}$ ont les significations indiquées précédemment.

**3.** Peptide conforme à la revendication 1, présentant la formule :

MEEEOC-Phe-Phe-Gly-Leu-Met-NH2

dans laquelle MEEEOC représente $CH_3$-O($CH_2$-$CH_2$-O)$_2$-$CH_2$-$CH_2$-OCO- et tous les acides aminés présentent la configuration L.

**4.** Peptide conforme à la revendication 1, présentant la formule :

MEEEOC-Gln-Phe-Phe-Gly-Leu-Met-NH2

dans laquelle MEEEOC a la signification indiquée précédemment et tous les acides aminés présentent la configuration L.

**5.** Peptide conforme à la revendication 1, présentant la formule :

MEEEOC-Gln-Gln-Phe-Phe-Gly-Leu-Met-NH2

dans laquelle MEEEOC a la signification indiquée précédemment et tous les acides aminés présentent la configuration L.

**6.** Peptide présentant la formule :

MEEEOC-Pro-Gln-Phe-Phe-Gly-Leu-Met-NH2

dans laquelle MEEEOC a la signification indiquée précédemment et tous les acides aminés présentent la configuration L.

**7.** Peptide conforme à la revendication 1, présentant la formule :

MEEEOC-Glu($\gamma$-OEEM)-Phe-Phe-Gly-Leu-Met-$NH_2$

dans laquelle MEEEOC a la signification indiquée précédemment, OEEM représente $CH_3$-$CH_2$-$CH_2$-O-$CH_2$-$CH_2$-O- et tous les acides aminés présentent la configuration L.

**8.** Peptide conforme à la revendication 1, présentant la formule :

MEEEOC-Gln-Glu-($\gamma$-OEEM)-Phe-Phe-Gly-Leu-Met-$NH_2$

dans laquelle MEEEOC et OEEM ont les significations indiquées précédemment et tous les acides aminés présentent la configuration L.

**9.** Peptide conforme à la revendication 1, présentant la formule :

MEEEOC-Pro-Gln-Glu-($\gamma$-OEEM)-Phe-Phe-Gly-Leu-Met-$NH_2$

dans laquelle MEEEOC et OEEM ont les significations indiquées précédemment et tous les acides aminés présentent la configuration L.

**10.** Peptide conforme à la revendication 1, présentant la formule :

MEEEOC-Glu-($\gamma$-OEEM)-Gln-Phe-Phe-Gly-Leu-Met-$NH_2$

dans laquelle MEEEOC et OEEM ont les significations indiquées précédemment et tous les acides aminés présentent la configuration L.

**11.** Peptide conforme à la revendication 1, présentant la formule :

MEEEOC-Pro-Glu-($\gamma$-OEEM)-Gln-Phe-Phe-Gly-Leu-Met-$NH_2$

dans laquelle MEEEOC et OEEM ont les significations indiquées précédemment et tous les acides aminés présentent la configuration L.

**12.** Peptide conforme à la revendication 1, présentant la formule :

MEEEOC-Glu-($\gamma$-OEEM)-Glu($\gamma$-OEEM)-Phe-Phe-Gly-Leu-Met-$NH_2$

dans laquelle MEEEOC et OEEM ont les significations indiquées précédemment et tous les acides aminés présentent la configuration L.

**13.** Peptide conforme à la revendication 1, présentant la formule :

MEEEOC-Pro-Glu-($\gamma$-OEEM)-Glu-($\gamma$-OEEM)-Phe-Phe-Gly-Leu-Met-$NH_2$

dans laquelle MEEEOC et OEEM ont les significations indiquées précédemment et tous les acides aminés présentent la configuration L.

**14.** Peptide conforme à la revendication 1, présentant la formule :

MEEEOC-Arg-Pro-Lys-Pro-Gln-Gln-Phe-Phe-Gly-Leu-Met-$NH_2$

dans laquelle MEEEOC et OEEM ont les significations indiquées précédemment et tous les acides aminés présentent la configuration L.

**15.** Peptide conforme à la revendication 1, présentant la formule :

MEEEOC-Arg-Pro-Lys-Pro-Glu-($\gamma$-OEEM)-Gln-Phe-Phe-Gly-Leu-Met-$NH_2$

dans laquelle MEEEOC et OEEM ont les significations indiquées précédemment et tous les acides aminés présentent la configuration L.

**16.** Peptide conforme à la revendication 1, présentant la formule :

MEEEOC-Arg-Pro-Lys-Pro-Gln-Glu-($\gamma$-OEEM)-Phe-Phe-Gly-Leu-Met-$NH_2$

dans laquelle MEEEOC et OEEM ont les significations indiquées précédemment et tous les acides aminés présentent la configuration L.

**17.** Peptide conforme à la revendication 1, présentant la formule :

H-Arg-Pro-Lys-($N^{\epsilon}$-MEEEOC)-Pro-Gln-Gln-Phe-Phe-Gly-Leu-Met-$NH_2$

dans laquelle MEEEOC a la signification indiquée précédemment et tous les acides aminés présentent la configuration L.

**18.** Peptide conforme à la revendication 1, présentant la formule :

H-Arg-Pro-Lys-($N^{\epsilon}$-MEEEOC)-Pro-Gln-Glu-($\gamma$-OEEM)-Phe-Phe-Gly-Leu-Met-$NH_2$

dans laquelle MEEEOC et OEEM ont les significations indiquées précédemment et tous les acides aminés présentent la configuration L.

**19.** Peptide conforme à la revendication 1, présentant la formule :

H-Arg-Pro-Lys-($N^{\epsilon}$-MEEEOC)-Pro-Glu-($\gamma$-OEEM)-Gln-Phe-Phe-Gly-Leu-Met-$NH_2$

dans laquelle MEEEOC et OEEM ont les significations indiquées précédemment et tous les acides aminés présentent la configuration L.

**20.** Peptide conforme à la revendication 1, présentant la formule :

H-Arg-Pro-Lys-($N^{\varepsilon}$-MEEEOC)-Pro-Glu-($\gamma$-OEEM)-Glu-($\gamma$-OEEM)-Phe-Phe-Gly-Leu-Met-$NH_2$

dans laquelle MEEEOC et OEEM ont les significations indiquées précédemment et tous les acides aminés présentent la configuration L.

**Revendications pour l'Etat contractant suivant: AT**

1. Procédé pour préparer des analogues de la substance P, représentés par formule générale :

$R\text{-}R^5\text{-}R^4\text{-}R^3\text{-}R^2\text{-}R^1\text{-}NH_2$ (I)

dont au moins un acide aminé porte, chimiquement lié, dans le cas d'un acide aminé acide, un substituant méthoxy-(éthoxy)$_n$-éthoxy, de formule générale :

$CH_3\text{-}O\text{-}(CH_2\text{-}CH_2\text{-}O)_n.CH_2\text{-}CH_2\text{-}O\text{-}$ (II)(MEE)

avec $\underline{n}$ = 0, 1, 2, 3,
et/ou, dans le cas d'un acide aminé basique, un substituant méthoxy-(éthoxy)$_n$-éthoxycarbonyle de formule générale :

$CH_3\text{-}O\text{-}(CH_2\text{-}CH_2\text{-}O)_n\text{-}CH_2\text{-}CH_2\text{-}O\text{-}CO\text{-}$ (III) (MEEEOC)

avec $\underline{n}$ = 0, 1, 2, 3,
dans laquelle :

$R^1$ est un résidu de méthionine, de méthionine-sulfoxyde, de méthionine-sulfone, ou de sélénométhionine,
$R^2$ est un résidu de leucine, de norleucine ou d'isoleucine,
$R^3$ est un résidu de glycine,
$R^4$ est un résidu de phénylalanine, de tryptophane ou de tyrosine,
$R^5$ est un résidu de phénylalanine, de tyrosine, de 4-chloro-phénylalanine, d'o-benzyltyrosine, ou de leurs dérivés à substituant acétyle, cyclopentyle, t-butyloxycarbonyle ou 4-hydroxyphényl-acétyle, et
R est choisi parmi :

a) un groupe présentant la formule générale (III) (MEEEOC) dans laquelle $\underline{n}$ vaut 0, 1, 2 ou 3 ;
b) un dérivé d'acide aminé, présentant la formule générale :

P-G (IV)

dans laquelle G est un résidu présentant la formule générale :

$$\begin{array}{l} -NH-CH-CO- \\ \quad\quad\;\; | \\ \quad\quad (CH_2)_s \\ \quad\quad\;\; | \\ \quad\quad COOCH_2-CH_2-(OCH_2-CH_2)_z-OCH_3 \end{array} \quad (V)$$

dans laquelle $\underline{s}$ vaut 1 ou 2 et z vaut 0, 1, 2 ou 3, et P représente un atome d'hydrogène, un groupe alkyle aliphatique linéaire ou ramifié, comportant de 1 à 6 atomes de carbone, ou un groupe acyle aliphatique linéaire ou ramifié, saturé ou non, choisi parmi formyle, acétyle, propionyle, n-butyryle, isobutyryle, n-valéryle, isovaléryle, hexanoyle, isohexanoyle, heptanoyle, octanoyle, crotonoyle, méthacryloyle, acryloyle, ou encore un groupe acyle substitué, choisi parmi hydroxyacétyle, 2-hydroxypropanoyle, 2-hydroxypropanoyle, 3-hydroxypropanoyle, aminoacétyle, 4-hydroxyphénylacétyle, 4-hydroxyphénylpropanoyle, 2-aminopropanoyle, 3-aminopropanoyle, o-éthylmalonyle, éthoxyformyle, méthoxyformyle, 3-méthoxypropanoyle, 3-éthoxypropanoyle, chloroacétyle, dichloro-acétyle, 2-chloropropanoyle, 3-chloropropanoyle, 2,3-dichloropropanoyle, bromoacétyle, 4-hydroxy-3,5-diiodophénylacétyle, 3-oxobutyryle, 3-oxovaléryle, méthylthioacétyle, 3-méthylthiopropanoyle, éthylthioacétyle, 3-éthylthiopropanoyle, nicotinoyle, 4-aminobutyryle, $N^{\alpha}$-[1-(9-adényl)-$\beta$-D-ribofuranuronosyle)], $N^{\alpha}$-[(1-(9-hypoxanthyl)-$\beta$-D-ribofuranuronosy-

le)] ou encore un groupe choisi parmi benzyloxycarbonyle, tertiobutyloxycarbonyle, tertioamyloxycarbonyle, isobornyloxycarbonyle, adamantyloxycarbonyle, benzyloxycarbonyle à substituant chloro ou nitro ;
c) un dérivé d'acide aminé présentant la formule générale :

$CH_3\text{-}O(CH_2\text{-}CH_2\text{-}O)_n\text{-}CH_2CH_2\text{-}O\text{-}CO\text{-}R^6$ (IV)

dans laquelle n vaut 0, 1, 2 ou 3 et $R^6$ représente un résidu de glutamine ou son dérivé MEEEOC, ou un résidu de formule (V) dans laquelle s et z sont tels que définis ci-dessus ;
d) un dérivé d'un segment dipeptidique présentant la formule générale :

$CH_3\text{-}O\text{-}(CH_2\text{-}CH_2\text{-}O)_n\text{-}CH_2\text{-}CH_2\text{-}O\text{-}CO\text{-}R^8\text{-}R^7$ (VII)

dans laquelle n vaut 0, 1, 2 ou 3, $R^7$ représente un résidu de glutamine ou un résidu de formule (V), dans laquelle s et z sont tels que définis ci-dessus, et $R^8$ représente un résidu de glutamine ou son dérivé MEEEOC, ou encore un résidu présentant la formule générale :

```
-NH-CH-CO-
    |
   (CH2)h
    |
   COO-CH2-CH2-(OCH2-CH2)k-O-CH3   (VIII)
```

dans laquelle h vaut 1 ou 2 et k vaut 0, 1, 2 ou 3 ;
e) un dérivé d'un segment dipeptidique présentant la formule générale :

$P\text{-}R^{10}\text{-}R^9$ (IX)

dans laquelle P présente la signification décrite précédemment, $R^9$ représente un résidu de formule (V), et $R^{10}$ représente un résidu de glutamine ou son dérivé MEEEOC, ou encore un résidu de formule (VIII), ou bien dans laquelle $R^{10}$ représente un résidu de formule (V) et $R^9$ représente un résidu de glutamine ou son dérivé MEEEOC, ou encore un résidu de formule (VIII) ;
f) un dérivé d'un segment tripeptidique présentant la formule générale :

$CH_3\text{-}O\text{-}(CH_2\text{-}CH_2\text{-}O)_n\text{-}CH_2\text{-}CH_2\text{-}O\text{-}CO\text{-}R^{11}\text{-}R^8\text{-}R^7$ (X)

dans laquelle n, $R^8$ et $R^7$ ont les significations indiquées précédemment, et $R^{11}$ représente un résidu de proline ;
g) un dérivé d'un segment tripeptidique présentant la formule générale :

$P\text{-}R^{11}\text{-}R^9\text{-}R^{10}$ (XI)

dans laquelle P, $R^9$, $R^{10}$ et $R^{11}$ ont les significations indiquées précédemment ;
h) un dérivé tétrapeptidique présentant la formule générale :

$CH_3\text{-}O(CH_2\text{-}CH_2\text{-}O)_n\text{-}CH_2\text{-}CH_2\text{-}O\text{-}CO\text{-}R^{12}\text{-}R^{11}\text{-}R^8\text{-}R^7$ (XII)

dans laquelle n, $R^7$, $R^8$ et $R^{11}$ ont les significations indiquées précédemment, et $R^{12}$ représente un résidu de lysine ou son dérivé MEEEOC, ou encore un résidu présentant la formule générale :

```
-NH-CH-CO-
    |
   (CH2)j
    |
   NH-CO-O-CH2-CH2-(O-CH2-CH2)i-OCH3          (XIII)
```

dans laquelle i vaut 0, 1, 2 ou 3 et j vaut 1, 2, 3 ou 4 ;
i) un dérivé tétrapeptidique présentant la formule générale :

$P-R^{12}-R^{11}-R^{10}-R^{9}$ (XIV)

dans laquelle P, $R^9$, $R^{10}$, $R^{11}$ et $R^{12}$ ont les significations indiquées précédemment ;
j) un dérivé pentapeptidique présentant la formule générale :

$CH_3-O(CH_2-CH_2-O-)_n-CH_2-CH_2-O-CO-R^{13}-R^{12}-R^{11}-R^{8}-R^{7}$ (XV)

dans laquelle n, $R^7$, $R^8$, $R^{11}$ et $R^{12}$ ont les significations indiquées précédemment, et $R^{13}$ représente un résidu de proline ;
k) un dérivé pentapeptidique présentant la formule générale :

$P-R^{13}-R^{12}-R^{11}-R^{10}-R^{9}$ (XVI)

dans laquelle P, $R^9$, $R^{10}$, $R^{11}$, $R^{12}$ et $R^{13}$ ont les significations indiquées précédemment ;
l) un dérivé hexapeptidique présentant la formule générale :

$CH_3-O(CH_2-CH_2-O)_n-CH_2-CH_2-O-CO-R^{14}-R^{13}-R^{12}-R^{11}-R^{8}-R^{7}$ (XVII)

dans laquelle n, $R^7$, $R^8$, $R^{11}$, $R^{12}$ et $R^{13}$ ont les significations indiquées précédemment, et $R^{14}$ représente un résidu d'arginine ou son dérivé MEEEOC ; ou
m) un dérivé hexapeptidique présentant la formule générale :

$P-R^{14}-R^{13}-R^{12}-R^{11}-R^{10}-R^{9}$ (XVIII)

dans laquelle P, $R^9$, $R^{10}$, $R^{11}$, $R^{12}$, $R^{13}$ et $R^{14}$ ont les significations indiquées précédemment,
**caractérisé** en ce que les groupes représentés par la formule (II) sont introduits dans la molécule de substance P sous forme d'esters ou de résidus de glutamine ou d'acide aspartique, tandis que les groupes représentés par la formule générale (III) sont introduits dans la molécule de substance P par réaction du chloroformiate de méthoxy-(éthoxy)$_n$-éthyle, de formule :

$CH_3-O(CH_2-CH_2-O)_nCH_2-CH_2-O-CO-Cl$

avec l'acide aminé choisi, après que l'on a convenablement protégé tous les autres groupes fonctionnels réactifs présents.

2. Procédé conforme à la revendication 1, **caractérisé** en ce que l'on prépare les esters d'acide glutamique ou d'acide aspartique avec des dérivés de l'acide aminé concerné, protégés au niveau des deux groupes $-NH_2$ et -COOH, par condensation des groupes carboxy libres avec du méthoxy-(éthoxy)-$_n$-éthanol, en présence de N,N-dicyclohexylcarbodiimide et de N,N-diméthylaminopyridine.

3. Procédé conforme à la revendication 1, **caractérisé** en ce que les dérivés d'acide aminé qui contiennent des groupes représentés par les formules (II) et (III) sont incorporés dans la chaîne polypeptidique par condensation avec du N,N-dicyclohexylcarbodiimide/hydroxybenzotriazole et activation de -COOH par des anhydrides mixtes, des anhydrides symétriques ou des esters actifs.

**Patentansprüche**
**Patentansprüche für folgende Vertragsstaaten: BE, CH, DE, FR, GB, LI, LU, NL, SE**

1. Analoga der "Substanz P", dargestellt durch die allgemeine Formel:

$R-R^5-R^4-R^3-R^2-R^1-NH_2$ (I),

worin wenigstens eine Aminosäure hievon,im Falle einer sauren Aminosäure, einen Methoxy(ethoxy)-$_n$ethoxy-Substituenten der allgemeinen Formel:

$CH_3$-O-($CH_2$-$CH_2$-O)$_n$-$CH_2$-$CH_2$-O- (II) (MEE)

mit n = 0, 1, 2 oder 3,und/oder, im Falle einer basischen Aminosäure, einen Methoxy(ethoxy)-$_n$ethoxycarbonyl-Substituenten der allgemeinen Formel:

$CH_3$-O-($CH_2$-$CH_2$-O-)$_n$-$CH_2$-$CH_2$-O-CO- (III) (MEEEOC)

mit n = 0, 1, 2 oder 3 chemisch gebunden trägt, worin

$R^1$ einen Rest von Methionin, Methioninsulfoxid, Methioninsulfon oder Selenomethionin bedeutet;
$R^2$ einen Rest von Leucin, Norleucin oder Isoleucin darstellt;
$R^3$ einen Glycinrest bedeutet;
$R^4$ einen Rest von Phenylalanin, Tryptophan oder Tyrosin bedeutet;
$R^5$ einen Rest von Phenylalanin, Tyrosin, 4-Chlorphenylalanin, o-Benzyltyrosin und den Acetyl-, Cyclopentyl-, tert.Butyloxycarbonyl- und 4-Hydroxyphenylacetylderivaten hievon darstellt und

R ausgewählt ist aus:

a) einer Gruppe mit der Formel (III)(MEEEOC), worin n = 0, 1, 2 oder 3 bedeutet;

b) einem Derivat einer Aminosäure mit der allgemeinen Formel:

P-G (IV),

worin G einen Rest mit der allgemeinen Formel:

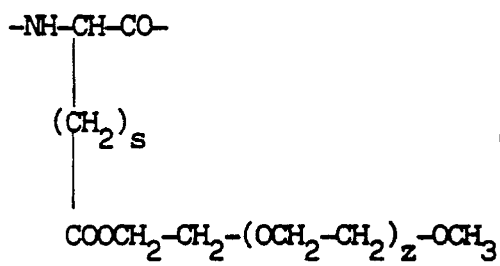

(V)

bedeutet, worin s den Wert 1 oder 2 aufweist und z den Wert 0, 1, 2 oder 3 hat, und P ein Wasserstoffatom, eine gerade oder verzweigte aliphatische Alkylgruppe mit 1 bis 6 Kohlenstoffatomen oder eine gerade oder verzweigte, gesättigte oder ungesättigte aliphatische Acylgruppe, ausgewählt unter Formyl, Acetyl, Propionyl, n-Butyryl, Isobutyryl, n-Valeryl, Isovaleryl, Hexanoyl, Isohexanoyl, Heptanoyl, Octanoyl,Crotonoyl, Methacryloyl, Acryloyl, oder eine substituierte Acylgruppe,ausgewählt unter Hydroxyacetyl, 2-Hydroxypropanoyl 2-Hydroxypropanoyl,3-Hydroxypropanoyl,Aminoacetyl,4-Hydroxyphenylacetyl, 4-Hydroxyphenylpropanoyl, 2-Aminopropanoyl, 3-Aminopropanoyl, o-Ethylmalonyl, Ethoxyformyl, Methoxyformyl, 3-Methoxypropanoyl, 3-Ethoxypropanoyl, Chloracetyl, Dichloracetyl, 2-Chlorpropanoyl, 3-Chlorpropanoyl, 2,3-Dichlorpropanoyl, Bromacetyl, 4-Hydroxy-3,5-diiodphenylacetyl, 3-Oxobutyryl, 3-Oxovaleryl, Methylthioacetyl, 3-Methylthiopropanoyl, Ethylthioacetyl, 3-Ethylthiopropanoyl, Nicotinoyl, 4-Aminobutyryl, $N^2$-[1-(9-Adenyl)-$\beta$-D-ribofuranuronosyl)], $N^2$-[(1-(9-Hypoxanthyl)-$\beta$-D-ribofuranuronosyl)] oder eine Gruppe, ausgewählt unter Benzyloxycarbonyl, tert.Butyloxycarbonyl, tert.Amyloxycarbonyl, Isobornyloxycarbonyl, Adamantyloxycarbonyl, Chlor- oder Nitro-substituiertem Benzyloxycarbonyl, bedeutet;

c) einem Aminosäurederivat der allgemeinen Formel:

$CH_3$-O($CH_2$-$CH_2$-O)$_n$-$CH_2$-$CH_2$-O-CO-$R^6$ (VI),

worin n den Wert 0, 1, 2 oder 3 hat und $R^6$ einen Rest von Glutamin und dem MEEEOC-Derivat hievon oder einen Rest der Formel (V) bedeutet, worin s und z wie oben definiert sind;

d) einem Derivat eines Dipeptidsegments mit der allgemeinen Formel

$CH_3$-O-($CH_2$-$CH_2$-O)$_n$-$CH_2$-$CH_2$-O-CO-$R^8$-$R^7$ (VII),

worin n den Wert 0, 1, 2 oder 3 hat, $R^7$ einen Glutaminrest oder einen Rest der Formel (V), worin s und z wie oben definiert sind, bedeutet und $R^8$ einen Glutaminrest oder den Rest des MEEEOC-Derivats hievon oder einen Rest mit der allgemeinen Formel:

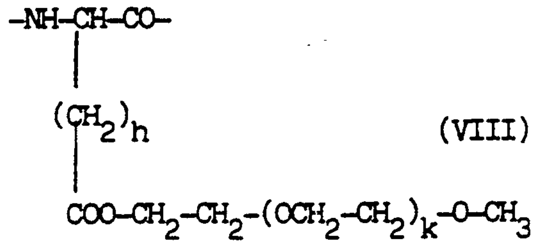

(VIII)

darstellt, worin h den Wert 1 oder 2 hat und k den Wert 0, 1, 2 oder 3 aufweist;
e) einem Derivat eines Dipeptidsegments mit der allgemeinen Formel

$P-R^{10}-R^{9}$ (IX),

worin P die zuvor angegebene Bedeutung hat, $R^{9}$ einen Rest der Formel (V) darstellt und $R^{10}$ einen Rest von Glutamin oder dem MEEEOC-Derivat hievon oder ein Derivat der Formel (VIII) bedeutet, oder worin $R^{10}$ einen Rest der Formel (V) darstellt und $R^{9}$ einen Rest von Glutamin oder dem MEEEOC-Derivat hievon darstellt oder einen Rest der Formel (VIII) bedeutet;
f) einem Derivat eines Tripeptidsegments mit der allgemeinen Formel

$CH_3-O-(CH_2-CH_2-O)_n-CH_2-CH_2-O-CO-R^{11}-R^{8}-R^{7}$ (X),

worin n, $R^{8}$ und $R^{7}$ die zuvor angegebenen Bedeutungen besitzen und $R^{11}$ einen Prolinrest darstellt;
g) einem Rest eines Tripeptidsegments mit der allgemeinen Formel:

$P-R^{11}-R^{9}-R^{10}$ (XI),

worin P, $R^{9}$, $R^{10}$ und $R^{11}$ die zuvor angegebenen Bedeutungen besitzen;
h) einem Tetrapeptidderivat mit der allgemeinen Formel

$CH_3-O(CH_2-CH_2-O-)_n-CH_2-CH_2-O-CO-R^{12}-R^{11}-R^{8}-R^{7}$ (XII),

worin n, $R^{7}$, $R^{8}$ und $R^{11}$ die zuvor angegebenen Bedeutungen aufweisen und $R^{12}$ einen Rest von Lysin oder dem MEEEOC-Derivat hievon bedeutet oder einen Rest mit der allgemeinen Formel:

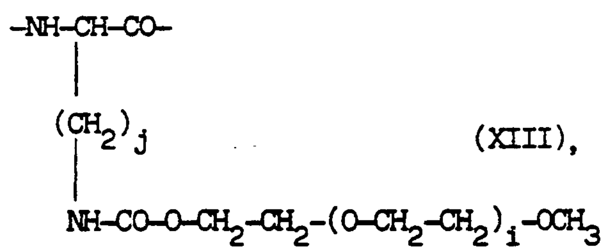

(XIII),

worin i den Wert 0, 1, 2 oder 3 hat und j den Wert 1, 2, 3 oder 4 aufweist;
i) einem Tetrapeptidderviat mit der allgemeinen Formel:

$P-R^{12}-R^{11}-R^{10}-R^{9}$ (XIV),

worin P, $R^{9}$, $R^{10}$, $R^{11}$ und $R^{12}$ wie zuvor definiert sind;
j) einem Pentapeptidderviat mit der allgemeinen Formel:

$CH_3-O(CH_2-CH_2-O-)_n-CH_2-CH_2-O-CO-R^{13}-R^{12}-R^{11}-R^{8}-R^{7}$ (XV),

worin n, $R^{7}$, $R^{8}$ $R^{11}$ und $R^{12}$ die zuvor angegebenen Bedeutungen besitzen und $R^{13}$ einen Prolinrest

darstellt;
k) einem Pentapeptidderivat mit der allgemeinen Formel

P-$R^{13}$-$R^{12}$-$R^{11}$-$R^{10}$-$R^9$ (XVI),

worin P, $R^9$, $R^{10}$, $R^{11}$, $R^{12}$ und $R^{13}$ die zuvor angegebenen Bedeutungen aufweisen;
l) einem Hexapeptidderviat der allgemeinen Formel:

$CH_3$-O($CH_2$-$CH_2$-O)$_n$-$CH_2$-$CH_2$-O-CO-$R^{14}$-$R^{13}$-$R^{12}$-$R^{11}$-$R^8$-$R^7$ (XVII),

worin n, $R^7$, $R^8$, $R^{11}$, $R^{12}$ und $R^{13}$ die zuvor angegebenen Bedeutungen besitzen und $R^{14}$ einen Rest von Arginin oder dem MEEEOC-Derivat hievon darstellt, oder
m) einem Hexapeptidderviat mit der allgemeinen Formel

P-$R^{14}$-$R^{13}$-$R^{12}$-$R^{11}$-$R^{10}$-$R^9$ (XVIII),

worin P, $R^9$, $R^{10}$, $R^{11}$, $R^{12}$, $R^{13}$ und $R^{14}$ die zuvor angegebenen Bedeutungen aufweisen.

**2.** Analoga von Substanz P nach Anspruch 1, worin R ausgewählt ist unter:
a) einer Gruppe der Formel (III) (MEEEOC);
b) einem Aminosäurederivat der Formel (IV), worin G einen Rest der Formel V bedeutet und P, s und z die zuvor angegebenen Bedeutungen besitzen;
c) einem Aminosäurederivat der Formel (VI), worin n und $R^6$ die zuvor angegebenen Bedeutungen aufweisen oder $R^6$ einen Rest der Formel (V) darstellt, worin s und z die zuvor angegebenen Bedeutungen besitzen;
d) einem Derviat eines Dipeptidsegments der Formel (VII), worin n die zuvor angegebene Bedeutung aufweist und $R^7$ einen Glutaminrest bedeutet oder einen Rest der Formel (V) darstellt, worin s und z die zuvor angegebenen Bedeutungen aufweisen, und $R^8$ einen Rest von Glutamin oder dem MEEEOC-Derivat hievon oder einen Rest der Formel (VIII) darstellt, worin h und k die zuvor angegebenen Bedeutungen besitzen;
e) einem Derivat eines Dipeptidsegments der Formel (IX), worin P die zuvor angegebenen Bedeutungen aufweist und $R^9$ einen Rest der Formel (V) darstellt, worin s und z die zuvor angegebenen Bedeutungen besitzen, und $R^{10}$ ein Rest von Glutamin oder dem MEEEOC-Derivat hievon oder ein Derivat der Formel (VIII) darstellt, worin h und k die zuvor angegebenen Bedeutungen besitzen, oder $R^{10}$ einen Rest der Formel (V) bedeutet, worin s und z die zuvor angegebenen Bedeutungen besitzen, und $R^9$ einen Rest von Glutamin oder dem MEEEOC-Derivat hievon oder einen Rest der Formel (VIII) darstellt, worin h und k die zuvor angegebenen Bedeutungen aufweisen;
f) einem Derivat eines Tripeptidsegments der Formel (X), worin n, $R^7$, $R^8$ und $R^{11}$ die zuvor angegebenen Bedeutungen aufweisen;
g) einem Derivat eines Tripeptidsegments der Formel (XI), worin P, $R^9$, $R^{10}$ und $R^{11}$ die zuvor angegebenen Bedeutungen besitzen;
h) einem Hexapeptidderivat der Formel (XVII), worin n, $R^7$, $R^8$, $R^{11}$, $R^{13}$ und $R^{14}$ die zuvor angegebenen Bedeutungen besitzen, und
i) einem Hexapeptidsegment der Formel (XVIII), worin P, $R^9$, $R^{10}$, $R^{11}$, $R^{12}$, $R^{13}$ und $R^{14}$ die zuvor angegebenen Bedeutungen aufweisen.

**3.** Peptid nach Anspruch 1 mit der Formel

MEEEOC-Phe-Phe-Gly-Leu-Met-$NH_2$,

worin MEEEOC für $CH_3$-O($CH_2$-$CH_2$-O)$_2$-$CH_2$-$CH_2$-OCO- steht und sämtliche Aminosäuren eine L-Konfiguration aufweisen.

**4.** Peptid nach Anspruch 1 mit der Formel:

MEEEOC-Gln-Phe-Phe-Gly-Leu-Met-$NH_2$,

worin MEEEOC die zuvor angegebene Bedeutung hat und alle Aminosäuren eine L-Konfiguration

aufweisen.

5. Peptid nach Anspruch 1 mit der Formel:

MEEEOC-Gln-Gln-Phe-Phe-Gly-Leu-Met-$NH_2$,

worin MEEEOC die zuvor angegebene Bedeutung hat und alle Aminosäuren eine L-Konfiguration aufweisen.

6. Peptid mit der Formel:

MEEEOC-Pro-Gln-Phe-Phe-Gly-Leu-Met-$NH_2$,

worin MEEEOC die zuvor angegebene Bedeutung hat und alle Aminosäuren eine L-Konfiguration aufweisen.

7. Peptid nach Anspruch 1 mit der Formel:

MEEEOC-Glu($\gamma$-OEEM)-Phe-Phe-Gly-Leu-Met-$NH_2$,

worin MEEEOC die zuvor angegebene Bedeutung hat, OEEM für $CH_3$-$CH_2$-$CH_2$-O-$CH_2$-$CH_2$-O- steht und alle Aminosäuren eine L-Konfiguration aufweisen.

8. Peptid nach Anspruch 1 mit der Formel:

MEEEOC-Gln-Glu($\gamma$–OEEM)-Phe-Phe-Gly-Leu-Met-$NH_2$,

worin MEEEOC und OEEM die zuvor angegebenen Bedeutungen haben und alle Aminosäuren eine L-Konfiguration aufweisen.

9. Peptid nach Anspruch 1 mit der Formel:

MEEEOC-Pro-Gln-Glu-($\gamma$-OEEM)-Phe-Phe-Gly-Leu-Met-$NH_2$,

worin MEEEOC und OEEM die zuvor angegebenen Bedeutungen haben und alle Aminosäuren eine L-Konfiguration aufweisen.

10. Peptid nach Anspruch 1 mit der Formel:

MEEEOC-Glu($\gamma$-OEEM)-Gln-Phe-Phe-Gly-Leu-Met-$NH_2$,

worin MEEEOC und OEEM die zuvor angegebenen Bedeutungen haben und alle Aminosäuren eine L-Konfiguration aufweisen.

11. Peptid nach Anspruch 1 mit der Formel:

MEEEOC-Pro-Glu-($\gamma$-OEEM)-Gln-Phe-Phe-Gly-Leu-Met-$NH_2$,

worin MEEEOC und OEEM die zuvor angegebenen Bedeutungen haben und alle Aminosäuren eine L-Konfiguration aufweisen.

12. Peptid nach Anspruch 1 mit der Formel:

MEEEOC-Glu($\gamma$-OEEM)-Glu ($\gamma$-OEEM)-Phe-Phe-Gly-Leu-Met-$NH_2$,

worin MEEEOC und OEEM die zuvor angegebenen Bedeutungen haben und alle Aminosäuren eine L-Konfiguration aufweisen.

13. Peptid nach Anspruch 1 mit der Formel:

MEEEOC-Pro-Glu-(γ--OEEM)-Glu-(γ--OEEM)-Phe-Phe-Gly-Leu-Met-$NH_2$,

worin MEEEOC und OEEM die zuvor angegebenen Bedeutungen haben und alle Aminosäuren eine L-Konfiguration aufweisen.

14. Peptid nach Anspruch 1 mit der Formel:

MEEEOC-Arg-Pro-Lys-Pro-Gln-Gln-Phe-Phe-Gly-Leu-Met-$NH_2$,

worin MEEEOC und OEEM die zuvor angegebenen Bedeutungen haben und alle Aminosäuren eine L-Konfiguration aufweisen.

15. Peptid nach Anspruch 1 mit der Formel:

MEEEOC-Arg-Pro-Lys-Pro-Glu--(γ-OEEM)-Gln-Phe-Phe-Gly-Leu-Met-$NH_2$,

worin MEEEOC und OEEM die zuvor angegebenen Bedeutungen haben und alle Aminosäuren eine L-Konfiguration aufweisen.

16. Peptid nach Anspruch 1 mit der Formel:

MEEEOC-Arg-Pro-Lys-Pro-Gln-Glu--(γ-OEEM)-Phe-Phe-Gly-Leu-Met-$NH_2$,

worin MEEEOC und OEEM die zuvor angegebenen Bedeutungen haben und alle Aminosäuren eine L-Konfiguration aufweisen.

17. Peptid nach Anspruch 1 mit der Formel:

H-Arg-Pro-Lys--(N$^ε$--MEEEOC)-Pro-Gln-Gln-Phe-Phe-Gly-Leu-Met-$NH_2$,

worin MEEEOC die zuvor angegebene Bedeutung hat und alle Aminosäuren eine L-Konfiguration aufweisen.

18. Peptid nach Anspruch 1 mit der Formel:

H-Arg-Pro-Lys(N$^ε$--MEEEOC)-Pro--Gln-Glu-(γ-OEEM)-Phe-Phe-Gly-Leu-Met-$NH_2$,

worin MEEEOC und OEEM die zuvor angegebenen Bedeutungen haben und alle Aminosäuren eine L-Konfiguration aufweisen.

19. Peptid nach Anspruch 1 mit der Formel:

H-Arg-Pro-Lys-(N$^ε$--MEEEOC)-Pro-Glu-(γ--OEEM)-Gln-Phe-Phe-Gly-Leu-Met-$NH_2$,

worin MEEEOC und OEEM die zuvor angegebenen Bedeutungen haben und alle Aminosäuren eine L-Konfiguration aufweisen.

20. Peptid nach Anspruch 1 mit der Formel:

H-Arg-Pro-Lys-(N$^ε$--MEEEOC)-Pro-Glu-(γ-OEEM)-Glu-(γ-OEEM)-Phe-Phe-Gly-Leu-Met-$NH_2$

worin MEEEOC und OEEM die zuvor angegebenen Bedeutungen haben und alle Aminosäuren eine L-Konfiguration aufweisen.

**Patentansprüche für folgenden Vertragsstaat: AT**

**1.** Verfahren zur Herstellung von Analoga von "Substanz P", dargestellt durch die allgemeine Formel:

$R\text{-}R^5\text{-}R^4\text{-}R^3\text{-}R^2\text{-}R^1\text{-}NH_2$ (I)

worin wenigstens eine Aminosäure hievon, im Falle einer sauren Aminosäure, einen Methoxy(ethoxy)-$_n$ethoxy-Substituenten der allgemeinen Formel:

$CH_3\text{-}O\text{-}(CH_2\text{-}CH_2\text{-}O)_n\text{-}CH_2\text{-}CH_2\text{-}O\text{-}$ (II) (MEE)

mit $\underline{n}$ = 0, 1, 2 oder 3, und/oder, im Falle einer basischen Aminosäure, einen Methoxy(ethoxy)-$_n$ethoxycarbonyl-Substituenten der allgemeinen Formel:

$CH_3\text{-}O\text{-}(CH_2\text{-}CH_2\text{-}O\text{-})_n\text{-}CH_2\text{-}CH_2\text{-}O\text{-}CO\text{-}$ (III) (MEEEOC)

mit n = 0, 1, 2 oder 3 chemisch gebunden trägt, worin

- $R^1$ einen Rest von Methionin, Methioninsulfoxid, Methioninsulfon oder Selenomethionin bedeutet;
- $R^2$ einen Rest von Leucin, Norleucin oder Isoleucin darstellt;
- $R^3$ einen Glycinrest bedeutet;
- $R^4$ einen Rest von Phenylalanin, Tryptophan oder Tyrosin bedeutet;
- $R^5$ einen Rest von Phenylalanin, Tyrosin, 4-Chlorphenylalanin, o-Benzyltyrosin und den Acetyl-, Cyclopentyl-, tert.Butyloxycarbonyl- und 4-Hydroxyphenylacetylderivaten hievon darstellt und

R ausgewählt ist aus:

a) einer Gruppe mit der Formel (III) (MEEEOC), worin $\underline{n}$ = 0, 1, 2 oder 3 bedeutet;

b) einem Derivat einer Aminosäure mit der allgemeinen Formel:

P-G (IV),

worin G einen Rest mit der allgemeinen Formel:

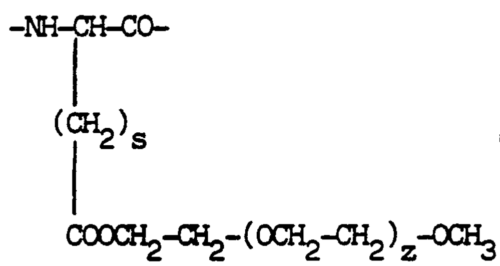

(V)

bedeutet, worin $\underline{s}$ den Wert 1 oder 2 aufweist und $\underline{z}$ den Wert 0, 1, 2 oder 3 hat, und P ein Wasserstoffatom, eine gerade oder verzweigte aliphatische Alkylgruppe mit 1 bis 6 Kohlenstoffatomen oder eine gerade oder verzweigte, gesättigte oder ungesättigte aliphatische Acylgruppe, ausgewählt unter Formyl, Acetyl, Propionyl, n-Butyryl, Isobutyryl, n-Valeryl, Isovaleryl, Hexanoyl, Isohexanoyl, Heptanoyl, Octanoyl,Crotonoyl, Methacryloyl, Acryloyl, oder eine substituierte Acylgruppe,ausgewählt unter Hydroxyacetyl, 2-Hydroxypropanoyl, 2-Hydroxypropanoyl,3-Hydroxypropanoyl,Aminoacetyl,4-Hydroxyphenylacetyl, 4-Hydroxyphenylpropanoyl, 2-Aminopropanoyl, 3-Aminopropanoyl, o-Ethylmalonyl, Ethoxyformyl, Methoxyformyl, 3-Methoxypropanoyl, 3-Ethoxypropanoyl, Chloracetyl, Dichloracetyl, 2-Chlorpropanoyl, 3-Chlorpropanoyl, 2,3-Dichlorpropanoyl, Bromacetyl, 4-Hydroxy-3,5-diiodphenylacetyl, 3-Oxobutyryl, 3-Oxovaleryl, Methylthioacetyl, 3-Methylthiopropanoyl, Ethylthioacetyl, 3-Ethylthiopropanoyl, Nicotinoyl, 4-Aminobutyryl, $N^2$-[1-(9-Adenyl)-ß-D-ribofuranuronosyl)], $N^2$-[(1-(9-Hypoxanthyl)-ß-D-ribofuranuronosyl)] oder eine Gruppe, ausgewählt unter Benzyloxycarbonyl, tert.Butyloxycarbonyl, tert.Amyloxycarbonyl, Isobornyloxycarbonyl, Adamantyloxycarbonyl, Chlor- oder Nitro-substituiertem Benzyloxycarbonyl, bedeutet;

c) einem Aminosäurederivat der allgemeinen Formel:

$CH_3\text{-}O(CH_2\text{-}CH_2\text{-}O)_n\text{-}CH_2\text{-}CH_2\text{-}O\text{-}CO\text{-}R^6$ (VI),

worin $\underline{n}$ den Wert 0, 1, 2 oder 3 hat und $R^6$ einen Rest von Glutamin und dem MEEEOC-Derivat

hievon oder einen Rest der Formel (V) bedeutet, worin s und z wie oben definiert sind;
d) einem Derivat eines Dipeptidsegments mit der allgemeinen Formel

$CH_3\text{-}O\text{-}(CH_2\text{-}CH_2\text{-}O)_n\text{-}CH_2\text{-}CH_2\text{-}O\text{-}CO\text{-}R^8\text{-}R^7$ (VII),

worin n den Wert 0, 1, 2 oder 3 hat, $R^7$ einen Glutaminrest oder einen Rest der Formel (V), worin s und z wie oben definiert sind, bedeutet und $R^8$ einen Glutaminrest oder den Rest des MEEEOC-Derivats hievon oder einen Rest mit der allgemeinen Formel:

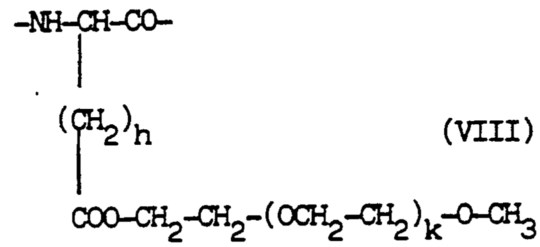

(VIII)

darstellt, worin h den Wert 1 oder 2 hat und k den Wert 0, 1, 2 oder 3 aufweist;
e) einem Derivat eines Dipeptidsegments mit der allgemeinen Formel

$P\text{-}R^{10}\text{-}R^9$ (IX),

worin P die zuvor angegebene Bedeutung hat, $R^9$ einen Rest der Formel (V) darstellt und $R^{10}$ einen Rest von Glutamin oder dem MEEEOC-Derivat hievon oder ein Derivat der Formel (VIII) bedeutet, oder worin $R^{10}$ einen Rest der Formel (V) darstellt und $R^9$ einen Rest von Glutamin oder dem MEEEOC-Derivat hievon darstellt oder einen Rest der Formel (VIII) bedeutet;
f) einem Derivat eines Tripeptidsegments mit der allgemeinen Formel

$CH_3\text{-}O\text{-}(CH_2\text{-}CH_2\text{-}O)_n\text{-}CH_2\text{-}CH_2\text{-}O\text{-}CO\text{-}R^{11}\text{-}R^8\text{-}R^7$ (X)

worin n, $R^8$ und $R^7$ die zuvor angegebenen Bedeutungen besitzen und $R^{11}$ einen Prolinrest darstellt;
g) einem Rest eines Tripeptidsegments mit der allgemeinen Formel:

$P\text{-}R^{11}\text{-}R^9\text{-}R^{10}$ (XI),

worin P, $R^9$, $R^{10}$ und $R^{11}$ die zuvor angegebenen Bedeutungen besitzen;
h) einem Tetrapeptidderivat mit der allgemeinen Formel

$CH_3\text{-}O(CH_2\text{-}CH_2\text{-}O\text{-})_n\text{-}CH_2\text{-}CH_2\text{-}O\text{-}CO\text{-}R^{12}\text{-}R^{11}\text{-}R^8\text{-}R^7$ (XII),

worin n, $R^7$, $R^8$ und $R^{11}$ die zuvor angegebenen Bedeutungen aufweisen und $R^{12}$ einen Rest von Lysin oder dem MEEEOC-Derivat hievon bedeutet oder einen Rest mit der allgemeinen Formel:

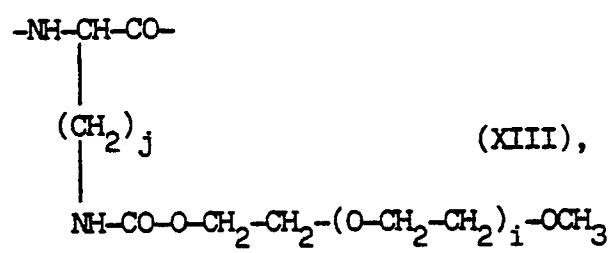

(XIII),

worin i den Wert 0, 1, 2 oder 3 hat und j den Wert 1, 2, 3 oder 4 aufweist;
i) einem Tetrapeptidderviat mit der allgemeinen Formel:

$P\text{-}R^{12}\text{-}R^{11}\text{-}R^{10}\text{-}R^9$ (XIV),

worin P, $R^9$, $R^{10}$, $R^{11}$ und $R^{12}$ wie zuvor definiert sind;
j) einem Pentapeptidderviat mit der allgemeinen Formel:

$CH_3$-O($CH_2$-$CH_2$-O-)$_n$-$CH_2$-$CH_2$-O-CO-$R^{13}$-$R^{12}$-$R^{11}$-$R^8$.$R^{78}$ (XV),

worin n, $R^7$, $R^8$ $R^{11}$ und $R^{12}$ die zuvor angegebenen Bedeutungen besitzen und $R^{13}$ einen Prolinrest darstellt;
k) einem Pentapeptidderivat mit der allgemeinen Formel

P-$R^{13}$-$R^{12}$-$R^{11}$-$R^{10}$-$R^9$ (XVI),

worin P, $R^9$, $R^{10}$, $R^{11}$, $R^{12}$ und $R^{13}$ die zuvor angegebenen Bedeutungen aufweisen;
l) einem Hexapeptidderviat der allgemeinen Formel:

$CH_3$-O($CH_2$-$CH_2$-O)$_n$-$CH_2$-$CH_2$-O-CO-$R^{14}$-$R^{13}$-$R^{12}$-$R^{11}$-$R^8$-$R^7$ (XVII),

worin n, $R^7$, $R^8$ $R^{11}$, $R^{12}$ und $R^{13}$ die zuvor angegebenen Bedeutungen besitzen und $R^{14}$ einen Rest von Arginin oder dem MEEEOC-Derivat hievon darstellt, oder
m) einem Hexapeptidderviat mit der allgemeinen Formel

P-$R^{14}$-$R^{13}$-$R^{12}$-$R^{11}$-$R^{10}$-$R^9$ (XVIII),

worin P, $R^9$, $R^{10}$, $R^{11}$ $R^{12}$, $R^{13}$ und $R^{14}$ die zuvor angegebenen Bedeutungen aufweisen,
dadurch gekennzeichnet, daß die durch die Formel (II) dargestellten Gruppen in das Molekül von Substanz P als Ester oder Reste von Glutamin- oder Aspareginsäure eingeführt werden, wogegen die durch die allgemeine Formel (III) dargestellten Gruppen durch Umsetzung von Methoxy(ethoxy)-$_n$ethylchlorcarbonat mit der Formel:

$CH_3$-O($CH_2$-$CH_2$-O)$_n$ $CH_2$-$CH_2$-O-CO-Cl

mit der ausgewählten Aminosäure nach entsprechendem Schutz aller anderen vorliegenden reaktiven funktionellen Gruppen in das Molekül von Substanz P eingeführt werden.

2. Verfahren nach Anspruch 1, dadurch gekennzeichnet, daß die Ester der Glutaminsäure oder Asparaginsäure mit Derivaten der betreffenden Aminosäure, geschützt sowohl an den $NH_2$- als auch den COOH-Gruppen, durch Kondensierenlassen der freien Carboxygruppen mit Methoxy(ethoxy)$_n$ethanol in Anwesenheit von N,N-Dicyclohexylcarbodiimid und N,N-Dimethylaminopyridin hergestellt werden.

3. Verfahren nach Anspruch 1 dadurch gekennzeichnet, daß die Aminosäurederivate, welche die durch die Formeln (II) und (III) dargestellten Gruppen enthalten, durch Kondensation mit N,N-Dicyclohexylcarbodiimid/Hydroxybenzotriazol und Aktivierung von -COOH durch gemischte Anhydride oder symmetrische Anhydride oder aktive Ester in eine Polypeptidkette eingeführt werden.